(19) Europäisches Patentamt
European Patent Office
Office européen des brevets

(11) Veröffentlichungsnummer: **0 001 051**
**B1**

(12) **EUROPÄISCHE PATENTSCHRIFT**

(21) Anmeldenummer: **78100621.8**

(22) Anmeldetag: **07.08.78**

(51) Int. Cl.³: **C 07 C 143/78**, C 07 D 207/26, C 07 D 295/22, A 61 K 31/18, A 61 K 31/40, C 07 D 405/12, C 07 D 317/28, C 07 D 319/06

# (54) Benzolsulfonamidderivate, Verfahren zu ihrer Herstellung und diese enthaltende pharmazeutische Präparate.

(30) Priorität: **18.08.77 DE 2737195**

(43) Veröffentlichungstag der Anmeldung:
**21.03.79 Patentblatt 79/6**

(45) Bekanntmachung des Hinweises auf die Patenterteilung:
**11.02.81 Patentblatt 81/6**

(84) Benannte Vertragsstaaten:
**BE CH DE FR GB NL SE**

(56) Entgegenhaltungen:
**DE - A - 2 139 072**
**DE - A - 2 145 686**
**DE - A - 2 321 518**
**DE - A - 2 453 356**
**DE - A - 2 533 821**
**DE - A - 2 756 783**

(73) Patentinhaber: **HOECHST AKTIENGESELLSCHAFT**
**Postfach 80 03 20**
**D-6230 Frankfurt/Main 80 (DE)**

(72) Erfinder: **Lang, Hans-Jochen, Dr.**
**Rüdesheimer Strasse 7**
**D-6238 Hofheim am Taunus (DE)**

(72) Erfinder: **Muschaweck, Roman, Dr.**
**Heimchenweg 39**
**D-6000 Frankfurt/MAin (DE)**

(72) Erfinder: **Hropot, Max, Dr.**
**Sossenheimer Weg 28**
**D-6000 Frankfurt/Main (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 051 B1

# Benzolsulfonamidderivate, Verfahren zu ihrer Herstellung und diese enthaltende pharmazeutische Präparate

Gegenstand der Erfindung sind Benzolsulfonamidderivate der allgemeinen Formel

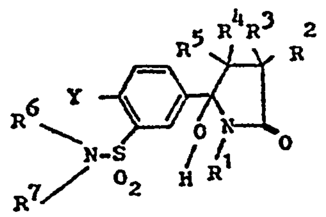

I

worin $R^1$ Wasserstoff, Alkyl mit 1—4 C-Atomen oder Alkenyl mit 2 bis 4 C-Atomen, wobei der Alkylrest auch eine Methoxygruppe tragen kann, Cycloalkyl mit 3 bis 5 Ringgliedern, Benzyl, $R^2$ bis $R^6$ Wasserstoff oder einen Alkylrest mit 1 bis 4 C-Atomen, $R^7$ Wasserstoff, Alkyl mit 1 bis 10 C-Atomen, wobei der Alkylrest auch 1 oder 2 Methoxy- oder Äthoxygruppen oder eine Äthylendioxy- oder eine Propylendioxygruppe tragen kann, Alkenyl mit 3 bis 5 C-Atomen, gegebenenfalls durch eine Methylgruppe substituiertes Cycloalkyl mit 3 bis 12 Ringgliedern, Cycloalkylalkyl mit 5 oder 6 Ringgliedern unt mit 1 oder 2 C-Atomen im Alkylteil, Phenylalkyl mit 1 bis 2 C-Atomen im Alkylteil, wobei der Phenylrest einfach oder zweifach substituiert sein und als Substituenten Methyl, Methoxy oder Chlor tragen kann, $R^6$ und $R^7$ auch gemeinsam mit dem N-Atom einen gesättigten 5 bis 6-gliedrigen heterocyclischen Ring, Y Halogen, Wasserstoff, Trifluoromethyl oder Methyl bedeutet, sowie die entsprechenden zu I offenkettigen tautomeren Formen der Formel I a

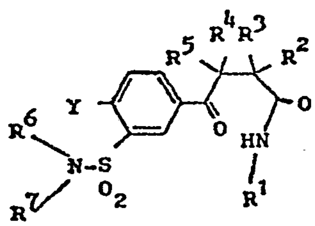

Ia

worin $R^1$ bis $R^7$ und Y die angegebene Bedeutung haben.

Gegenstand der Erfindung ist weiterhin ein Verfahren zur Herstellung der Verbindungen der allgemeinen Formel I, das dadurch gekennzeichnet ist, daß man

a) Verbindungen der allgemeinen Formel II

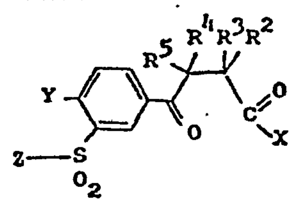

II

worin Z ein Halogenatom oder die $NR^6R^7$-Gruppe und X Halogen, Alkoxy, gegebenenfalls substituiertes Phenoxy, Nitril, Azid, einen aktivierten Esterrest oder den Rest eines gemischten Anhydrids bedeutet und $R^2$ bis $R^7$ sowie Y die in Anspruch 1 angegebene Bedeutung besitzen, mit einem Amin der Formel III

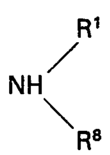

III

worin $R^1$ die in Anspruch 1 angegebene Bedeutung hat und $R^8$ Wasserstoff oder einen Benzyl- oder Diphenylmethylrest bedeutet, die gegebenenfalls durch Methoxygruppen substituiert sein können, umsetzt und, falls $R^8$ nicht Wasserstoff bedeutet, die erhaltenen Verbindungen der Hydrogenolyse oder Hydrolyse unterwirft oder

b) Verbindungen der allgemeinen Formel IV

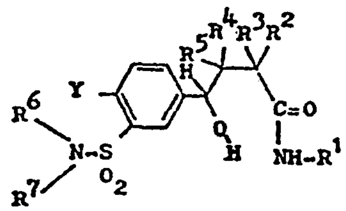

IV

worin $R^1$ bis $R^7$ und Y die angegebene Bedeutung besitzen, mit einem Oxidationsmittel behandelt, oder c) Verbindungen der allgemeinen Formel

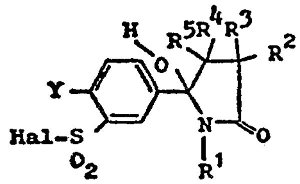

V

worin Y und $R^1$ bis $R^5$ die obige Bedeutung besitzen und Hal für Halogen steht, mit Ammoniak oder einem Amin der allgemeinen Formel VI

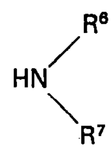

VI

worin $R^6$ und $R^7$ die angegebene Bedeutung besitzen, umsetzt oder d) Verbindungen der allgemeinen Formel VII

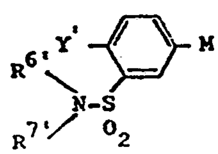

VII

worin M für Li oder MgHal steht, Y' die Bedeutung von Y hat, jedoch nicht für Brom oder Jod steht, und $R^{6\prime}$ und $R^{7\prime}$ mit Ausnahme von Wasserstoff die Bedeutung von $R^6$ und $R^7$ besitzen oder für ein Metallkation stehen, mit Succinimidderivaten der allgemein Formel

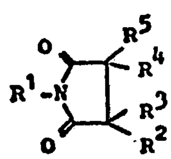

VIII

worin $R^1$ bis $R^5$ die angegebene Bedeutung besitzen, umsetzt und die erhaltenen Verbindungen nachfolgend mit einer Säure behandelt, oder e) Verbindungen der allgemeinen Formel IX

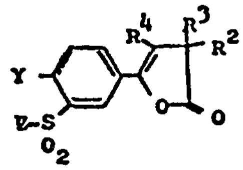

IX

mit einem Amin der Formel III umsetzt, wobei $R^1$ bis $R^4$, Z und Y die angegebene Bedeutung besitzen und gegebenenfalls die nach Weg a) bis e) erhaltenen Verbindungen der allgemeinen Formel I, worin $R^6$ und/oder $R^7$ Wasserstoff bedeutet, alkyliert.

Die Verbindungen der Formel I und V können auch in ihren offenkettigen tautomeren Formen I a) und V a) vorliegen:

I 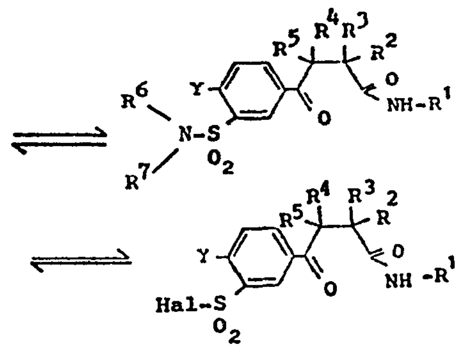 Ia

V Va

In den Ausführungsbeispielen werden beide möglichen automeren Formen angegeben.

Die erfindungsgemäßen Verbindungen der Formel I können außerdem in ihren geometrischen isomeren Strukturen vorliegen.

Die Alkyl- bzw. Alkenylreste in den Substituenten $R^1$ bis $R^7$ können sowohl geradkettig wie verzweigt sein.

In den für Verfahrensweise a) als Ausgangsstoffe verwendeten Verbindungen der Formel II bedeutet X als "leaving group" Halogen, insbesondere Chlor, einen Alkoxy- oder gegebenenfalls substituierten Phenoxyrest, eine Nitril-, Azid-gruppe oder einen aktivierten Esterrest wie den Cyanomethoxyrest $-OCH_2CN$. Von besonderem Vorteil ist jedoch der Rest eines gemischten Anhydrids.

Die Herstellung dieser Verbindungen II erfolgt in üblicher Weise aus den Carbonsäuren entsprechend der Formel II (X = OH). Die gemischten Anhydride werden durch Umsetzung entsprechender Carbonsäuresalze (X in Formel II z.B. ONa, OK, O-Trialkylammonium) mit einem aktivierten Säurederivat erhalten. Hierfür verwendet man beispielsweise einen Chlorameisensäurealkylester, bevorzugt Chlorameisensäureäthylester oder Chlorameisensäuremethylester, ein Carbamidsäurechlorid wie beispielsweise N,N-Dimethylcarbamidsäurechlorid oder N,N-Diäthylcarbamidsäurechlorid oder das Säurechlorid einer aromatischen oder aliphatischen Sulfonsäure, wie beispielsweise Methan, Äthan-, Benzol- oder p-Toluolsulfonsäurechlorid. Die Umsetzung zum gemischten Anhydrid führt man vorteilhaft etwa bei −30° bis +30°C in einem wasserfreien polaren organischen Lösungsmittel, wie beispielsweise Aceton, Methyläthylketon, Essigester, Dimethylformamid, Dimethylacetamid, Dimethylsulfoxid, Acetonitril, einem niederen aliphatischen Alkohol oder bevorzugt in Tetrahydrofuran, Dioxan, Äthylenglykoldimethyläther oder entsprechenden Glykoläthern durch.

Obgleich die gemischten Anhydride isoliert werden können, indem man beispielsweise das Lösungsmittel bei Temperaturen zwischen −5° und +10°C verdampft und z.B. mit Essigsäureäthylester aus dem Rückstand extrahiert, ist es vorteilhaft, ohne Isolierung des gemischten Anhydrides das. Amin der allgemeinen Formel III zum Reaktionsgemisch zuzufügen. Das Amin kann sowohl unverdünnt oder in einem der genannten Lösungsmittel zugegeben werden, es können auch wäßrige Lösungen des Amins oder von Ammoniak verwendet werden. Dabei kommt mindestens 1 Mol, vorteilhaft aber ein Überschuß (10 fach und mehr) an Amin zur Anwendung. Die Reaktion wird in einem Temperaturbereich zwischen +30°C und +100°C, bevorzugt zwischen +5°C und +40°C durchgeführt. Die Reaktionszeit liegt zwischen 10 Minuten und 5 Tagen, die Reaktion wird zweckmäßig dünnschichtchromatografisch, vorteilhaft auf Kieselgel, verfolgt.

Die aus den Carbonsäuren der Formel II (X = OH) mit $COCl_2$, Oxalylchlorid, $POCl_3$, $SOCl_2$, $PCl_3$ oder $PI_5$ darstellbaren Säurechloride der Formel II, worin X für Chlor steht, werden prinzipiell wie die gemischten Anhydride mit einem Amin der Formel III umgesetzt.

Bedeutet X in Verbindungen der allgemeinen Formel II eine niedere Alkoxygruppe mit 1 bis 6 C-Atomen, bevorzugt 1—4 C-Atomen, oder einen gegebenenfalls durch Halogen substituierten Phenoxyrest, wird die Umsetzung mit einem Amin der Formel III in Wasser oder einem polaren, gegenüber Aminen inerten organischen Lösungsmittel, beispielsweise in einem niederen Alkansäureamid, wie Dimethylformamid, Dimethylacetamid, in Dimethylsulfoxid, Dimethylsulfon, in einem ringförmigen oder offenkettigen Äther oder Polyäther, wie beispielsweise Tetrahydrofuran, Dioxan, Äthylenglykoldimethyläther, Äthylenglykoldiäthyläther, Diäthylenglykoldimethyläther, Diäthylenglykoldiäthyläther, bevorzugt aber in einem niederen Aklohol, wie beispielsweise in Methanol, Äthanol, Propanol, Isopropanol oder in überschüssigem Amin durchgeführt. Man arbeitet innerhalb eines Temperaturbereiches von 0°C bis 120°C, bevorzugt zwischen 10°C und 60°C, insbesondere zwischen 15°C und 30°C. Der Reaktionsverlauf wird durch Dünnschichtchromatogramm, z.B. an Kieselgel mit Methanol, Essigester, Toluol oder Gemischen dieser Lösungsmittel als Laufmittel kontrolliert. Die Reaktionsdauer beträgt zwischen 1 Stunde und 14 Tagen, beispielsweise bei Zimmertemperatur zwischen 5 und 72 Stunden. Die Carbonsäureester der Formel II und die Amine der Formel III werden bevorzugt in einem Molverhältnis von 1 : 1 bis 1 : 3 umgesetzt; das Amin kann auch in einem bis zu 10 fach molaren Überschuß angewendet werden.

Die Verbindungen der Formel II, worin X für einen Alkoxyrest steht, erhält man wie üblich durch

Umsetzung der Carbonsäuren II (X = OH) mit einem niederen Alkanol mit 1—6 C-Atomen, z.B. in Gegenwart eines organischen oder anorganischen Säurechlorids.

In gleicher Weise wie die Ester lassen sich ebenfalls die entsprechenden Säurecyanide, Säureazide, oder aktivierten Ester der Formel II mit X = CN, $N_3$, —O—$CH_2$CN mit einem Amin der Formel III zur Reaktion bringen.

Bei der Aufarbeitung nach Aminolyse verfährt man vorteilhaft so, daß man unter vermindertem Druck Lösungsmittel und überschüssiges Amin ganz oder teilweise entfernt, gegebenenfalls wieder Wasser zusetzt und mit einer organischen oder anorganischen Säure, wie HCl, Essigsäure, Ammoniumchlorid auf einem pH zwischen 0 und 12, bevorzugt zwischen 5 und 10 bringt. Der Niederschlag wird abfiltriert, wobei er gegebenenfalls zuvor durch übliche Operationen zur Kristallisation gebracht werden muß, oder mit einem geeigneten Lösungsmittel wie Essigester extrahiert, die Lösung getrocknet und nach dem Einengen zur Kristallisation gebracht.

Die Amine der Formel III sind literaturbekannt. Die Carbonsäuren der Formel II mit X = OH, worin Z ein Halogenatom bedeutet, (vergleiche Formel XIII) gewinnt man beispielsweise durch Friedel-Crafts-Reaktion einer aromatischen Verbindung X mit einem Bernsteinsäureanhydrid XI zur Verbindung XII

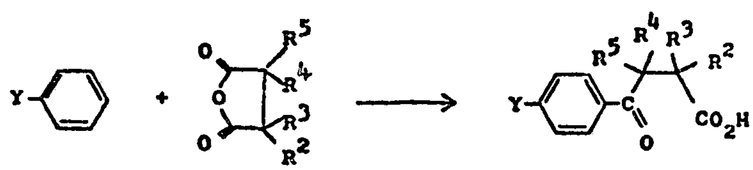

X XI XII

durch anschließende Nitrierung, Reduktion, Diazotierung und Meerwein-Reaktion erhält man Verbindung XIII

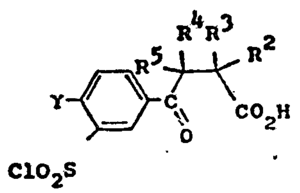

XIII

Die Überführung der Verbindung XIII in die entsprechende Sulfamoylverbindung der Formel II (Z = $NR^6R^7$, X = OH) wird durch Umsetzung von XIII mit Ammoniak oder einem Amin Formeln III bzw. $NHR^6R^7$ wie üblich erreicht.

Wenn die Umsetzung mit einem Amin der Formel III, in der $R^8$ nicht Wasserstoff bedeutet, erfolgt, werden die erhaltenen Verbindungen anschließend der Hydrolyse oder Hydrogenolyse unterworfen. Die Hydrogenolyse wird angewendet, wenn $R^8$ den Benzylrest bedeutet. Dann wird in üblicher Weise katalytisch hydriert. Man arbeitet bei einer Temperatur zwischen 0°C und 60°C, bevorzugt zwischen 10°C und 30°C mit Wasserstoffgas unter Atmosphärendruck oder leichtem Überschuß und vorteilhaft mit Palladium oder bevorzugt mit Palladium auf Tierkohle als Katalysator, wobei man die Reaktion nach Erreichen der theoretischen $H_2$-Aufnahme abbricht.

Wenn $R^8$ den Diphenylmethylrest oder einen substituierten Benzyl- oder Diphenylmethylrest, der im Kern eine oder mehrere Methoxygruppen enthält, bedeutet, wird dieser Rest hydrolytisch, vorzugsweise im sauren Medium acidolytisch, gespalten. Um eine Polymerenbildung oder andere Nebenreaktionen zu unterbinden, setzt man "Kationenfänger", bevorzugt die äquimolekulare Menge Anisol zum Reaktionsgemisch zu. Als Säure verwendet man vorzugsweise Trifluoressigsäure oder wasserfreien flüssigen Fluorwasserstoff. Man arbeitet zwischen 0 und 70°C, vorzugsweise zwischen 10 und 40°C; wobei die Reaktionsdauer zwischen 1 Stunde und 5 Tagen liegt. (Houben-Weyl, "Methoden der Organischen Chemie, Synthese von Peptiden Teil I", 1974, S. 265 und 461—463). Man arbeitet wie oben auf.

Als Oxidationsmittel gemäß Verfahrensweise b) kommen vorzugsweise aktives Mangan-IV-oxid, Eisen-III-salze, Chrom-VI-verbindungen, oder Cer-IV-salze in Betracht. Als Lösungsmittel verwendet man halogenierte Kohlenwasserstoffe, wie z.B. Methylenchlorid, Chloroform, Tetrachloräthan, halogenierte Derivate des Benzols wie Chlorbenzol, Fluorbenzol, Benzotrifluorid, vorzugsweise aber Acetonitril, wobei man die Reaktion bei Temperaturen zwischen 0°C bis 40°C, vorzugsweise zwischen 20°C und 30°C, über eine Dauer von 1 bis 60 Stunden, vorzugsweise über eine Dauer von 6 bis 36 Stunden durchführt.

Die Ausgangsstoffe der Formel IV erhält man beispielsweise dadurch, daß man Lactone der Formel XIV

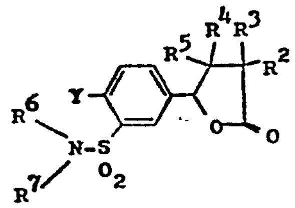

XIV

in üblicher Weise mit Aminen der Formel III aufspaltet. Dabei setzt man 1 Mol des Lactons mit überschüssigem Ammoniak bzw. Amin III, gegebenenfalls verdünnt in einem polaren Lösungsmittel, vorzugsweise einem niederen Alkohol, bei einer Temperatur zwischen 20 und 200°C um.

Die Lactone der Formel XIV erhält man beispielsweise aus Carbonsäuren der Formel II (X = OH und Z = $NR^6R^7$) durch Reduktion mit $NaBH_4$.

Gemäß Verfahrensweise c) werden Sulfonsäurechloride der allgemeinen Formel V mit Ammoniak oder einem Amin der Formel VI umgesetzt. Dabei können sowohl wäßrige Lösungen von Ammoniak und der Amine VI wie auch flüssiges Ammoniak bzw. reine Amine im Überschuß verwendet werden, wobei das überschüssige Ammoniak bzw. Amin gleichzeitig als Lösungsmittel fungiert. Die Reaktion wird vorteilhaft auch in polaren organischen Lösungsmitteln, wie beispielsweise Dimethylformamid, Dimethylsulfoxid, Dioxan, Tetrahydrofuran, Diäthylenglykoldimethyläther, bevorzugt aber in niederen Alkoholen mit 1—4 C-Atomen, wie z.B. in Methanol, Äthanol, Isopropanol durchgeführt. Man bringt pro Mol Sulfochlorid V mindestens 2 Mole Ammoniak oder Amin zur Anwendung, vorteilhaft jedoch einen größeren Überschuß. Man kann auch mit einem Mol Ammoniak oder Amin VI arbeiten, wenn die Reaktion in Anwesenheit einer Hilfsbase durchgeführt wird, wobei etwa 1—6 Moläquivalente Hilfsbase verwendet werden. Als Hilfsbasen eignen sich organische oder anorganische Hydroxide, Carbonate, Hydrogencarbonate, sowie Salzlösungen schwacher organischer und anorganischer Säuren, Tertiäre Amine, wie beispielsweise Triäthylamin, Tri-n-butylamin oder Äthyldicyclohexylamin sind besonders vorteilhaft, gegebenenfalls arbeitet man in Gegenwart eines der genannten Lösungsmittel oder deren Gemisch.

Man arbeitet bei Temperaturen zwischen —30 und +80°C, bevorzugt zwischen +10° und +35°C. Die Reaktionszeit liegt zwischen 6 und 20 Stunden. Die Aufarbeitung erfolgt wie oben.

Die Verbindungen der Formel V, bei denen Hal bevorzugt für Chlor steht, werden aus Verbindungen der Formel XV, worin E eine Nitro- oder Aminogruppe bedeutet, hergestellt. Man setzt entsprechende reaktionsfähige Carbonsäurederivate, in denen OH durch X ersetzt ist, mit Aminen der Formel III zu den Verbindungen der Formel XVI

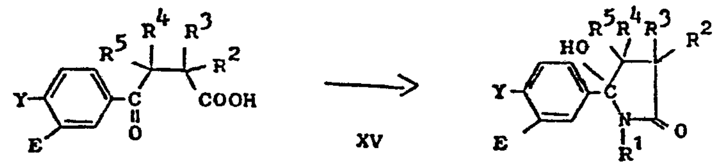

XV

XVI

worin E eine $NO_2$— oder $NH_2$-Gruppe bedeutet um. Falls E einen $NO_2$-Rest bedeutet, wird er zur Aminogruppe reduziert. Aus den Aminoverbindungen der Formel XVI werden durch Diazotierung und nachfolgende Meerwein-Reaktion die Sulfochloride der Formel V erhalten.

Gemäß Verfahrensweise d) bringt man Verbindungen der Formel VII, worin $R^6$ und $R^7$ zweckmäßig nicht für Wasserstoff steht, mit Succinimiden der allgemeinen Formel VIII zur Reaktion. Die Verbindungen VII und VIII werden vorteilhaft im Molverhältnis 1 : 1 bis 1 : 1,5 in einem für metallorganische Reaktionen üblichen inerten wasserfreien Lösungsmittel umgesetzt. Vorzugsweise verwendet man als Lösungsmittel ringförmige oder offenkettige Äther oder Polyäther, wie beispielsweise Diäthyläther, Tetrahydrofuran, Dioxan, Äthylenglykoldimethyläther oder Diäthylenglykoldimethyläther. Man arbeitet in einem Temperaturbereich zwischen 0 und 100°C, vorzugsweise zwischen 15 und 50°C, die Reaktionsdauer beträgt zwischen 1 und 30 Stunden. Vorteilhaft gibt man die Lösung oder Suspension von 1 Mol der metallorganischen Verbindung VII zu einer Lösung von 1 bis 1,5 Mol der Verbindungen VIII in einem der angegebenen Lösungsmittel in kleinen Portionen zu. Nach Beendigung der Umsetzung werden werden die Reaktionsprodukte in einer für metallorganische Reaktionen üblichen Weise hydrolysiert, beispielsweise wird das Reaktionsgemisch bei Temperaturen zwischen —5° und +20°C unter Aufrechterhaltung eines pH-Bereiches von 6 bis 9 in eine wäßrige, gesättigte Ammonchlorid-Lösung eingetragen. Die weitere Aufarbeitung der so erhaltenen Verbindungen der Formel I erfolgt wie beschrieben.

Die Verbindungen der allgemeinen Formel VII erhält man in einer für die Darstellung metallorganischer Reagenzien ublichen Weise z.B. durch Umsetzung von Verbindungen der Formel VII, worin M beispielsweise Br oder J bedeutet, mit Li oder Magnesium oder einer metallorganischen Verbindung der genannten Metalle [Houben-Weyl, Methoden der Organischen Chemie, 13/1 (1070), S. 134—159; Band 13/2a (1973), S.54—162].

Die Verbindung der Formel VII mit M = Br erhält man beispielsweise aus Verbindungen der allgemeinen Formel XVII

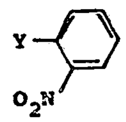

XVII

durch Bromierung, anschließende Reduktion der Nitrogruppe. Die erhaltene Aminogruppe wird diazotiert, nach Meerwein sulfochloriert und schließlich mit einem Amin der Formel VI umgesetzt. Die Verbindungen der Formel VII, worin $R^{6'}$ und/oder $R^{7'}$ ein Metallkation bedeutet, erhält man aus den entsprechenden Verbindungen VII mit $R^{6'}$ und/oder $R^{7'}$ = Wasserstoff und M = Br oder J durch Umsetzung mit einer Metallbase, beispielsweise einem Metallhydroxid oder einer metallorganischen Verbindung und nachfolgender Metallierung von Brom oder J in der oben beschriebenen Weise.

Gemäß Verfahrensweise e) verfährt man vorteilhaft so, daß man Verbindungen der allgemeinen Formel IX mit Aminen der Formel III zu Verbindungen der Formel I umsetzt. Die Reaktionsbedingungen, d.h. Reaktionstemperatur, Reaktionsdauer, angewandte Reagenzien und Solvenzien entsprechen den obigen Aminumsetzungen. Die Verbindungen der allgemeinen Formel IX gewind man aus den Carbonsäuren der Formel II (X = OH) durch Einwirkung eines wasserabspaltenden Mittels, vorzugsweise Thionylchlorid oder Acetanhydrid. Bei Verwendung von Acetanhydrid werden solche Verbindungen der Formel IX bevorzugt, bei denen $R^6$ und $R^7$ nicht für Wasserstoff steht, um eine ungewünschte Acylierung zu vermeiden.

Die Alkylierung der Sulfonamidgruppe in Verbindungen der allgemeinen Formel I, worin $R^6$ und/oder $R^7$ Wasserstoff bedeutet, erfolgt in üblicher Weise, beispielsweise mittels Formaldehyd/Ameisensäure, Alkylhalogeniden, Dialkylsulfat, Alkyltosylaten oder Alkylmesylaten in üblicher Weise.

Verbindungen der Formel I, worin $R^1$ nicht Methyl bedeutet, sind dann als bevorzugt anzusehen, wenn $R^6$ und $R^7$ für Wasserstoff stehen, insbesondere dann, wenn $R^2$ bis $R^5$ Wasserstoff bedeutet.

Als besonders bevorzugte Verbindungen kommen Verbindungen der Formel I in Betracht, worin $R^1$ Methyl und Y Chlor oder Brom bedeutet, wovon wiederum jene Verbindungen als besonders bevorzugt gelten, bei denen $R^2$ bis $R^5$ für Wasserstoff steht.

Die Verfahrensprodukte sind wertvolle Arzneimittel und zeichnen sich durch eine sehr gute diuretische und saluuretische Wirksamkeit aus.

Es ist bekannt, daß 4-(4-Chlor-3-sulfamoylphenyl)-4-oxobutansäure sowie ihr Methylester eine mäßige salidiuretische Wirksamkeit an der Ratte zeigen (Arzneimittel-Forsch. *13*, 269 (1963)).

Weiterhin ist bekannt, daß 1-Alkyl-5-hydroxy-5-aryl-2-oxopyrrolidine, die keine Sulfonamidgruppe am Arylrest tragen, ZNS-stimulierende Wirkungen zeigen (DOS 24 53 356, US—Pat. 3 947 460).

Es war nun überraschend, daß die erfindungsgemäßen Amid-Derivate I bzw. I a eine sehr starke salidiuretische Wirkung besitzen, die den aufgeführten bekannten Produkten in quantitativer und qualitativer Hinsicht deutlich überlegen sind. Darüber hinaus zeigen einige der erfindungsgemäßen Verbindungen an der Oxonat behandelten Ratte in einer Einheitsdosis von 50 mg/kg im Gegensatz zu den meisten salidiuretisch wirksamen Arzneimitteln eine für die therapeutische Anwendung vorteilhaft zu bewertende deutliche Erhöhung der Harnsäureausscheidung und der Harnsäure-Clearance.

Die salidiuretische Wirkung der neuen Verfahrensprodukte wurde an der Ratte in einer Einheitsdosis von 50 mg/kg per os bestimmt. Sie übertreffen dabei die salidiuretische Aktivität bekannter Handelspräparate der Thiazidgruppe, wie beispielsweise des Hydrochlorthiazids, und die des Chlorthalidons. Darüber hinaus zeichnen sich die neuen Verfahrenserzeugnisse durch eine lang anhaltende Wirkungsdauer aus. Deshalb sind die neuen Verfahrensprodukte insbesondere zur Behandlung hypertoner Zustände beim Menschen geeignet, wobei man sie, wie heute allgemein üblich, gegebenenfalls mit einem Antihypertonikum kombinieren wird.

Als therapeutische Zubereitung der neuen Verbindungen kommen vor allem Tabletten, Dragees, Kapseln, Suppositorien sowie auch Ampullen zur parenteralen Verabreichung (i.v., s.c. und i.m.) in Frage. Die therapeutische Einheitsdosis liegt zwischen 0,5 und 500 mg, vorzugsweise 10 bis 100 mg pro Tablette. Als Tagesdosis wird im allgemeinen eine Tablette täglich verabreicht.

Diese Zubereitungen können speziell bei der Behandlung des Bluthochdrucks außer den üblichen Füll- und Trägerstoffen noch ein Antihypertensivum, wie beispielsweise Reserpin, Hydralazin, Guanethidin, $\alpha$-Methyldopa, Clonidin oder einen $\beta$-sympathikolytischen Wirkstoff, wie beispielsweise Propanolol, enthalten.

Außerdem sind therapeutische Kombinationspräparate mit kaliumretinierenden Verbindungen, wie Aldosteronantagonisten, z.B. Spironolacton oder Pseudoaldosteronantagonisten wie Triamteren oder Amilorid von Interesse. Weiterhin kommt $K^+$-Substitution in verschiedenen Anwendungsformen, z.B. Dragees, Tabletten, Brausetabletten, Säften u.a. in Frage.

Von therapeutischem Interesse können ebenfalls Kombinationen der erfindungsgemäßen Verbindungen mit einem anderen antihyperurikämisch wirksamen Mittel sein, das besonders über eine Hemmung der Xanthinoxidase zu einer Verstärkung der antiurikopathischen Effekte führt.

In den nachfolgenden Beispielen sind die Schmelz- und Zersetzungspunkte der Ausführungsbeispiele nicht korrigiert.

Erfindungsgemäß können außer den in den Ausführungsbeispielen beschriebenen Benzolsulfonamidderivaten beispielsweise auch die in der folgenden Tabelle zusammengestellten Verbindungen der allgemeinen Formel I bzw. I a erhalten werden.

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | $-C_2H_5$ | Cl |
| $CH_3$ | H | H | H | H | H | $-CH(CH_3)_2$ | Cl |
| $CH_3$ | H | H | H | H | H | $-C(CH_3)_3$ | Cl |
| $CH_3$ | H | H | H | H | H | $-(CH_2)_3CH_3$ | Cl |
| $CH_3$ | H | H | H | H | H | $-(CH_2)_3CH(CH_3)_2$ | Cl |
| $CH_3$ | H | H | H | H | H | $-(CH_2)_4CH_3$ | Cl |
| $CH_3$ | H | H | H | H | H | $CH_3CH-(CH_2)_2CH_3$ | Cl |
| $CH_3$ | H | H | H | H | H | $-CH(CH_2CH_3)_2$ | Cl |
| $CH_3$ | H | H | H | H | H | $-(CH_2)_6CH_3$ | Cl |
| $CH_3$ | H | H | H | H | H | $-(CH_2)_8CH_3$ | Cl |
| $CH_3$ | H | H | H | H | H | $-(CH_2)_9CH_3$ | Cl |
| $CH_3$ | H | H | H | H | H | $-(CH_2)_2-OCH_3$ | Cl |
| $CH_3$ | H | H | H | H | H | $-(CH_2)_3-OCH_3$ | Cl |
| $CH_3$ | H | H | H | H | H | $-CH_2-CH(OCH_3)-CH_2(OCH_3)$ | Cl |
| $CH_3$ | H | H | H | H | H | $-CH_2-CH(OCH_3)_2$ | Cl |
| $CH_3$ | H | H | H | H | H | $-(CH_2)_2-CH(OCH_3)_2$ | Cl |
| $CH_3$ | H | H | H | H | H | $-(CH_2)_3-CH(OCH_3)_2$ | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 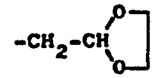 | Cl |
| $CH_3$ | H | H | H | H | H | 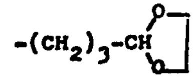 | Cl |
| $CH_3$ | H | H | H | H | H | 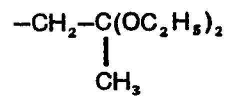 | Cl |
| $CH_3$ | H | H | H | H | H | 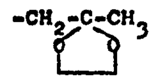 | Cl |
| $CH_3$ | H | H | H | H | H | 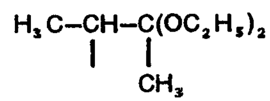 | Cl |
| $CH_3$ | H | H | H | H | H | 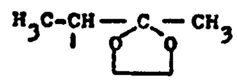 | Cl |
| $CH_3$ | H | H | H | H | H | 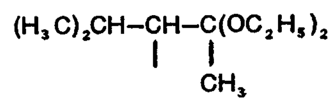 | Cl |
| $CH_3$ | H | H | H | H | H | 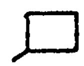 | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 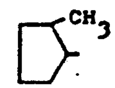 | Cl |
| $CH_3$ | H | H | H | H | H | 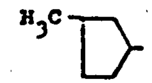 | Cl |
| $CH_3$ | H | H | H | H | H | 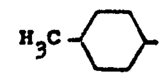 | Cl |
| $CH_3$ | H | H | H | H | H | 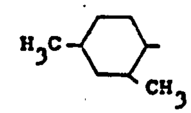 | Cl |
| $CH_3$ | H | H | H | H | H | 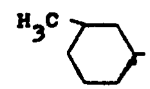 | Cl |
| $CH_3$ | H | H | H | H | H | 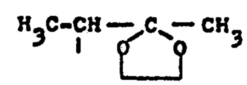 | Cl |

| R¹ | R² | R³ | R⁴ | R⁵ | R⁶ | R⁷ | Y |
|---|---|---|---|---|---|---|---|
| $CH_3$ | H | H | H | H | H | $(H_3C)_2CH-CH-C(OC_2H_5)_2$<br>\| \|<br>$CH_3$ | Cl |
| $CH_3$ | H | H | H | H | H | 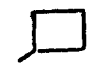 | Cl |
| $CH_3$ | H | H | H | H | H | 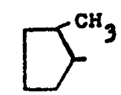 | Cl |
| $CH_3$ | H | H | H | H | H | 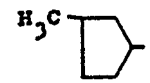 | Cl |
| $CH_3$ | H | H | H | H | H | 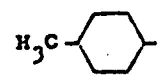 | Cl |
| $CH_3$ | H | H | H | H | H | 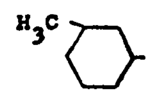 | Cl |
| $CH_3$ | H | H | H | H | H | 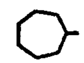 | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 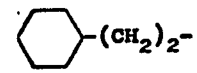 | Cl |
| $CH_3$ | H | H | H | H | H | 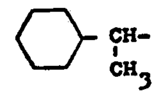 | Cl |
| $CH_3$ | H | H | H | H | H | 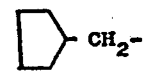 | Cl |
| $CH_3$ | H | H | H | H | H | 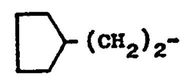 | Cl |
| $CH_3$ | H | H | H | H | H | 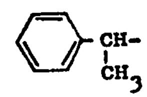 | Cl |
| $CH_3$ | H | H | H | H | H | 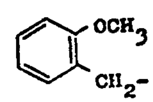 | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 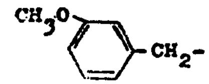 | Cl |
| $CH_3$ | H | H | H | H | H | 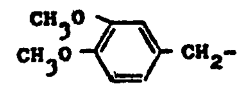 | Cl |
| $CH_3$ | H | H | H | H | H | 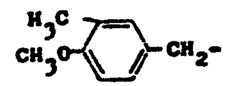 | Cl |
| $CH_3$ | H | H | H | H | H | 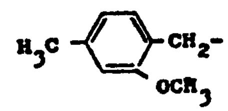 | Cl |
| $CH_3$ | H | H | H | H | H | 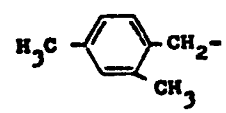 | Cl |
| $CH_3$ | H | H | H | H | H | 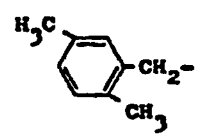 | Cl |

| R¹ | R² | R³ | R⁴ | R⁵ | R⁶ | R⁷ | Y |
|---|---|---|---|---|---|---|---|
| $CH_3$ | H | H | H | H | H | 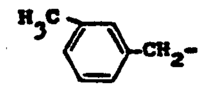 | Cl |
| $CH_3$ | H | H | H | H | H | 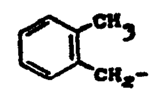 | Cl |
| $CH_3$ | H | H | H | H | H | 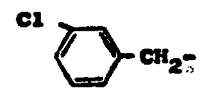 | Cl |
| $CH_3$ | H | H | H | H | H | 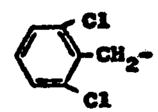 | Cl |
| $CH_3$ | H | H | H | H | H | 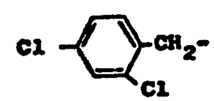 | Cl |
| $CH_3$ | H | H | H | H | H | 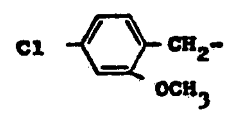 | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 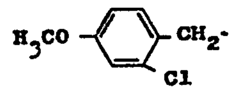 | Cl |
| $CH_3$ | H | H | H | H | H | $CH_3$ | Br |
| $CH_3$ | H | H | H | H | H | $(CH_2)_2CH_3$ | Br |
| $C_2H_5$ | H | H | H | H | H | H | Br |
| 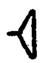 | H | H | H | H | H | H | Br |
| $-CH_2-CH-CH_3$<br>$O-CH_3$ | H | H | H | H | H | H | Br |
| $CH_2=CH-CH_2-$ | H | H | H | H | H | H | Br |
| $CH_3$ | H | H | H | H | H | $-(CH_2)_3-CH_3$ | Br |
| H | H | H | H | H | H | H | Br |
| $CH_3$ | H | H | H | H | H | $-CH-CH_2-CH_3$<br>$CH_3$ | Br |
| $CH_3$ | H | H | H | H | H | $-(CH_2)_5-CH_3$ | Br |
| $CH_3$ | H | H | H | H | H | $-(CH_2)_7-CH_3$ | Br |
| $CH_3$ | H | H | H | H | H | $-CH_2-CH=CH_2$ | Br |
| $CH_3$ | H | H | H | H | H | $-CH_2-CH-CH_3$<br>$O-CH_3$ | Br |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | $-CH_2CH(OC_2H_5)_2$ | Br |
| $CH_3$ | H | H | H | H | H | 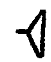 | Br |
| $CH_3$ | H | H | H | H | H | 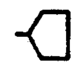 | Br |
| $CH_3$ | H | H | H | H | H | 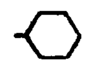 | Br |
| $CH_3$ | H | H | H | H | H | 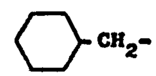 | Br |
| $CH_3$ | H | H | H | H | H | 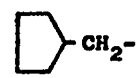 | Br |
| $CH_3$ | H | H | H | H | H | 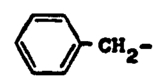 | Br |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 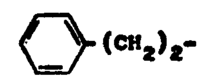 | Br |
| $CH_3$ | H | H | H | H | H | 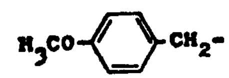 | Br |
| $CH_3$ | H | H | H | H | H | 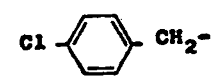 | Br |
| $CH_3$ | H | H | H | H | H | 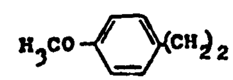 | Br |
| $CH_3$ | H | H | H | H | H | 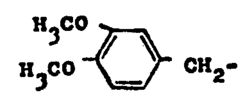 | Br |
| $CH_3$ | H | H | H | H | H | 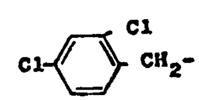 | Br |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 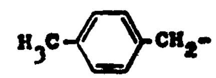 | Br |
| $CH_3$ | H | H | H | H | $C_2H_5$ | $C_2H_5$ | Cl |
| $CH_3$ | H | H | H | H | $-(CH_2)_3$<br>$CH_3$ | $-(CH_2)_3CH_3$ | Cl |
| $CH_3$ | H | H | H | H | $CH_3$ | 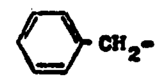 | Cl |
| $CH_3$ | H | H | H | H | $CH_3$ | $-CH_2-CH-CH_3$<br>$OCH_3$ | Cl |
| $CH_3$ | H | H | H | H | $CH_3$ | 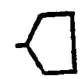 | Cl |
| $CH_3$ | H | H | H | H | $CH_3$ | $-CH_2-CH=CH_2$ | Cl |
| $CH_3$ | H | H | H | H | $CH_3$ | 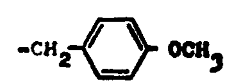 | Cl |
| $CH_3$ | H | H | H | H | $CH_3$ | 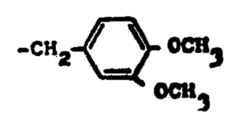 | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | $CH_3$ | 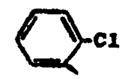 | Cl |
| $CH_3$ | H | H | H | H | $CH_3$ | $-(CH_2)_3-CH_3$ | Cl |
| $CH_3$ | H | H | H | H | $CH_3$ | $-CH_2-CH(CH_3)_2$ | Cl |
| $CH_3$ | H | H | H | H | $CH_3$ | $CH_3$ | Br |
| $CH_3$ | H | H | H | H | $(CH_2)_2-$<br>$CH_3$ | $-(CH_2)_2CH_3$ | Br |
| $CH_3$ | H | H | H | H | $(CH_2)_3$<br>$CH_3$ | $-(CH_2)_3CH_3$ | Br |
| H | H | H | H | H | H | H | $CF_3$ |
| $CH_3$ | H | H | H | H | H | H | $CF_3$ |
| $CH_3$ | H | H | H | H | H | $CH_3$ | $CF_3$ |
| $CH_3$ | H | H | H | H | H | $-(CH_2)_3CH_3$ | $CF_3$ |
| $CH_3$ | H | H | H | H | H | $CH_3-CH-CH_2CH_3$ | $CF_3$ |
| $CH_3$ | H | H | H | H | $CH_3$ | $CH_3$ | $CF_3$ |
| $CH_3$ | H | H | H | H | $(CH_2)_2-$<br>$CH_3$ | $-(CH_2)_2-CH_3$ | $CF_3$ |
| $CH_3$ | H | H | H | H | H | $-(CH_2)_5-CH_3$ | $CF_3$ |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | $CH_2-CH=CH_2$ | $CF_3$ |
| $CH_3$ | H | H | H | H | H | 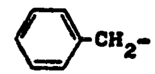 | $CF_3$ |
| $CH_3$ | H | H | H | H | H | $-CH_2-CH(OC_2H_5)_2$ | $CF_3$ |
| 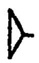 | H | H | H | H | H | H | $CF_3$ |
| $CH_2-CH-CH_3$<br>$OCH_3$ | H | H | H | H | H | H | $CF_3$ |
| H | H | H | H | H | H | H | H |
| $CH_3$ | H | H | H | H | H | H | H |
| $CH_3$ | H | H | H | H | H | $CH_3$ | H |
| $CH_3$ | H | H | H | H | H | $CH_2CH_3$ | H |
| $CH_3$ | H | H | H | H | H | $(CH_2)_2CH_3$ | H |
| $CH_3$ | H | H | H | H | H | $(CH_2)_3CH_3$ | H |
| $CH_3$ | H | H | H | H | H | $CH_3-CH-CH_2CH_3$ | H |
| $CH_3$ | H | H | H | H | H | $-(CH_2)_5CH_3$ | H |
| $CH_3$ | H | H | H | H | H | 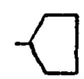 | H |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | 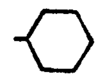 | H |
| $CH_3$ | H | H | H | H | H | 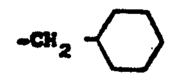 | H |
| $CH_3$ | H | H | H | H | H | 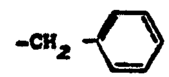 | H |
| $CH_3$ | H | H | H | H | H | 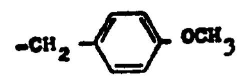 | H |
| H | H | H | H | H | H | H | $CH_3$ |
| $CH_3$ | H | H | H | H | H | H | $CH_3$ |
| $CH_3$ | H | H | H | H | H | $CH_3$ | $CH_3$ |
| $CH_3$ | H | H | H | H | H | $C_2H_5$ | $CH_3$ |
| $CH_3$ | H | H | H | H | H | $-(CH_2)_2CH_3$ | $CH_3$ |
| $CH_3$ | H | H | H | H | H | $-(CH_2)_3CH_3$ | $CH_3$ |
| $CH_3$ | H | H | H | H | H | $H_3C-CH-CH_2-CH_3$ | $CH_3$ |

| $R^1$ | $R^2$ | $R^3$ | $R^4$ | $R^5$ | $R^6$ | $R^7$ | Y |
|---|---|---|---|---|---|---|---|
| $CH_3$ | H | H | H | H | H | $-(CH_2)_5-CH_3$ | $CH_3$ |
| $CH_3$ | H | H | H | H | H | 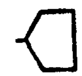 | $CH_3$ |
| $CH_3$ | H | H | H | H | H | 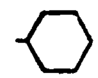 | $CH_3$ |
| $CH_3$ | H | H | H | H | H | $-CH_2-$ 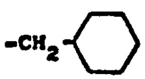 | $CH_3$ |
| $CH_3$ | H | H | H | H | H | $-CH_2-CH(OC_2H_5)_2$ | $CH_3$ |
| $CH_3$ | H | H | H | H | H | $CH_2$ 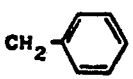 | $CH_3$ |
| $CH_3$ | H | H | H | H | H | $CH_3O$ 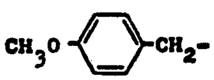 $CH_2-$ | $CH_3$ |
| H | $CH_3$ | H | H | H | H | H | Cl |
| $CH_3$ | $CH_3$ | H | H | H | H | H | Cl |

| $R^1$ | $R^2$ | $R^3$ | $R^4$ | $R^5$ | $R^6$ | $R^7$ | Y |
|---|---|---|---|---|---|---|---|
| $CH_3$ | $CH_3$ | H | H | H | H | $CH_3$ | Cl |
| $CH_3$ | $CH_3$ | H | H | H | H | $-(CH_2)_2CH_3$ | Cl |
| $CH_3$ | $CH_3$ | H | H | H | H | $-(CH_2)_3CH_3$ | Cl |
| $CH_3$ | $CH_3$ | H | H | H | H | $CH_3-CH-CH_2CH_3$ | Cl |
| $CH_3$ | $CH_3$ | H | H | H | H | $-(CH_2)_5CH_3$ | Cl |
| $CH_3$ | $CH_3$ | H | H | H | H | 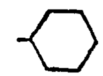 | Cl |
| $CH_3$ | $CH_3$ | H | H | H | H | $-CH_2CH(OC_2H_5)_2$ | Cl |
| $CH_3$ | $CH_3$ | H | H | H | H | 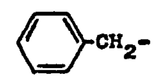 | Cl |
| $CH_3$ | $CH_3$ | H | H | H | $CH_3$ | $CH_3$ | Cl |
| $CH_3$ | $CH_3$ | H | H | H | $(CH_2)_2-$<br>$CH_3$ | $-(CH_2)_2-CH_3$ | Cl |
| H | H | H | $CH_3$ | H | H | H | Cl |
| $CH_3$ | H | H | $CH_3$ | H | H | H | Cl |
| $CH_3$ | H | H | $CH_3$ | H | H | $CH_3$ | Cl |
| $CH_3$ | H | H | $CH_3$ | H | H | $-(CH_2)_2CH_3$ | Cl |
| $C_2H_5$ | H | H | $CH_3$ | H | H | H | Cl |
| $CH_3$ | H | H | $CH_3$ | H | H | $CH_3CH-CH_2CH_3$ | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | $CH_3$ | H | H | $-(CH_2)_5CH_3$ | Cl |
| $CH_3$ | H | H | $CH_3$ | H | H | $-(CH_2)_7CH_3$ | Cl |
| $CH_3$ | H | H | $CH_3$ | H | H | 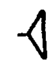 | Cl |
| $CH_3$ | H | H | $CH_3$ | H | H | 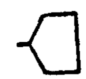 | Cl |
| $CH_3$ | H | H | $CH_3$ | H | H | 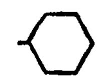 | Cl |
| $CH_3$ | H | H | $CH_3$ | H | H | 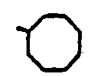 | Cl |
| $CH_3$ | H | H | $CH_3$ | H | H | $-CH_2-CH(OCH_3)-CH_3$ | Cl |
| $CH_3$ | H | H | $CH_3$ | H | H | $-CH_2(OC_2H_5)_2$ | Cl |
| $CH_3$ | H | H | $CH_3$ | H | H | $-(CH_2)_3CH_3$ | Cl |

| $R^1$ | $R^2$ | $R^3$ | $R^4$ | $R^5$ | $R^6$ | $R^7$ | Y |
|---|---|---|---|---|---|---|---|
| $CH_3$ | H | H | $CH_3$ | H | H | 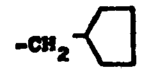 | Cl |
| $CH_3$ | H | H | $CH_3$ | H | H | 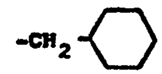 | Cl |
| $CH_3$ | H | H | $CH_3$ | H | H | 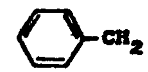 | Cl |
| $CH_3$ | H | H | $CH_3$ | H | H | 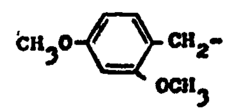 | Cl |
| $CH_3$ | H | H | $CH_3$ | H | H | 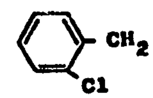 | Cl |
| $CH_3$ | H | H | $CH_3$ | H | H | 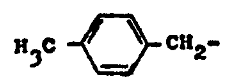 | Cl |
| H | H | H | $CH_3$ | H | H | H | Br |

| $R^1$ | $R^2$ | $R^3$ | $R^4$ | $R^5$ | $R^6$ | $R^7$ | Y |
|---|---|---|---|---|---|---|---|
| $CH_3$ | H | H | $CH_3$ | H | H | H | Br |
| $CH_3$ | H | H | $CH_3$ | H | H | $CH_3$ | Br |
| $CH_3$ | H | H | $CH_3$ | H | H | $-(CH_2)_3CH_3$ | Br |
| $CH_3$ | H | H | $CH_3$ | H | $CH_3$ | $CH_3$ | Cl |
| $CH_3$ | H | H | $CH_3$ | H | $-(CH_2)_2CH_3$ | $-(CH_2)_2CH_3$ | Cl |
| H | $CH_3$ | H | $CH_3$ | H | H | H | Cl |
| $CH_3$ | $CH_3$ | H | $CH_3$ | H | H | H | Cl |
| $CH_3$ | $CH_3$ | H | $CH_3$ | H | H | H | Br |
| $CH_3$ | $CH_3$ | H | $CH_3$ | H | H | $CH_3$ | Cl |
| $CH_2-CH=CH_2$ | $CH_3$ | H | $CH_3$ | H | H | H | Cl |
| $CH_3$ | $CH_3$ | H | $CH_3$ | H | $CH_3$ | $CH_3$ | Cl |
| $CH_3$ | $CH_3$ | H | $CH_3$ | H | H | $CH_3-CH-CH_2-CH_3$ | Cl |
| $CH_3$ | $CH_3$ | H | $CH_3$ | H | H | $-(CH_2)_3CH_3$ | Cl |
| H | H | H | $CH_3$ | $CH_3$ | H | H | Cl |
| $CH_3$ | H | H | $CH_3$ | $CH_3$ | H | H | Cl |
| $-C_2H_5$ | H | H | $CH_3$ | $CH_3$ | H | H | Cl |
| $-CH(CH_3)_2$ | H | H | $CH_3$ | $CH_3$ | H | H | Cl |
| $-CH_2-CH=CH_2$ | H | H | $CH_3$ | $CH_3$ | H | H | Cl |

| $R^1$ | $R^2$ | $R^3$ | $R^4$ | $R^5$ | $R^6$ | $R^7$ | Y |
|---|---|---|---|---|---|---|---|
| 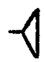 | H | H | $CH_3$ | $CH_3$ | H | H | Cl |
| $CH_3$ | H | H | $CH_3$ | $CH_3$ | H | $CH_3$ | Cl |
| $CH_3$ | H | H | $CH_3$ | $CH_3$ | H | $(CH_2)_3CH_3$ | Cl |
| $CH_3$ | H | H | $CH_3$ | $CH_3$ | $CH_3$ | $CH_3$ | Cl |
| $CH_3$ | H | H | $CH_3$ | $CH_3$ | $-(CH_2)_2CH_3$ | $-(CH_2)_2CH_3$ | Cl |
| $CH_3$ | H | H | $CH_3$ | $CH_3$ | H | $-CH_2$ 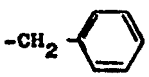 | Cl |
| $CH_3$ | H | H | $CH_3$ | $CH_3$ | H | H | Br |
| $CH_3$ | H | H | $CH_3$ | $CH_3$ | H | H | H |
| $CH_3$ | H | H | $CH_3$ | $CH_3$ | H | 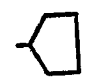 | Cl |
| H | $CH_3$ | $CH_3$ | H | H | H | H | Cl |
| $CH_3$ | $CH_3$ | $CH_3$ | H | H | H | H | Cl |
| $C_2H_5$ | $CH_3$ | $CH_3$ | H | H | H | H | Cl |
| H | $CH_3$ | $CH_3$ | H | H | H | H | Cl |
| $CH_3$ | $CH_3$ | $CH_3$ | H | H | H | $CH_3$ | Cl |
| $CH_3$ | $CH_3$ | $CH_3$ | H | H | H | $-(CH_2)_3CH_3$ | Cl |
| $CH_3$ | $CH_3$ | $CH_3$ | H | H | H | $-(CH_2)_5CH_3$ | Cl |

| $R^1$ | $R^2$ | $R^3$ | $R^4$ | $R^5$ | $R^6$ | $R^7$ | Y |
|---|---|---|---|---|---|---|---|
| $CH_3$ | $CH_3$ | $CH_3$ | H | H | H | 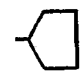 | Cl |
| $CH_3$ | $CH_3$ | $CH_3$ | H | H | H | 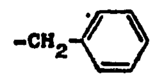 | Cl |
| $CH_3$ | $CH_3$ | $CH_3$ | H | H | $CH_3$ | $CH_3$ | Cl |
| $CH_3$ | $CH_3$ | $CH_3$ | H | H | $-(CH_2)_2CH_3$ | $-(CH_2)_2CH_3$ | Cl |
| $CH_3$ | H | H | $C_2H_5$ | H | H | H | Cl |
| $CH_3$ | H | H | $C_2H_5$ | H | H | $CH_3$ | Cl |
| $CH_3$ | H | H | $C_2H_5$ | H | H | $-(CH_2)_2CH_3$ | Cl |
| $CH_3$ | H | H | $C_2H_5$ | H | H | $-(CH_2)_5CH_3$ | Cl |
| $CH_3$ | H | H | $C_2H_5$ | H | $-(CH_2)_2CH_3$ | $-(CH_2)_2CH_3$ | Cl |
| $CH_3$ | $C_2H_5$ | H | H | H | H | H | Cl |
| $CH_3$ | $C_2H_5$ | H | H | H | H | $CH_3$ | Cl |
| $CH_3$ | $C_2H_5$ | H | H | H | H | $-(CH_2)_3CH_3$ | Cl |
| $CH_3$ | $C_2H_5$ | H | H | H | H | 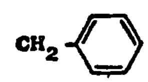 | Cl |
| $CH_3$ | $C_2H_5$ | H | H | H | $-(CH_2)_2CH_3$ | $-(CH_2)_2CH_3$ | Cl |

| $R^1$ | $R^2$ | $R^3$ | $R^4$ | $R^5$ | $R^6$ | $R^7$ | Y |
|---|---|---|---|---|---|---|---|
| $CH_3$ | $C_2H_5$ | $C_2H_5$ | H | H | H | H | Cl |
| $CH_3$ | H | H | $-(CH_2)_2CH_3$ | H | H | H | Cl |
| $CH_3$ | H | H | $-CH(CH_3)_2$ | H | H | H | Cl |
| $CH_3$ | H | H | $-(CH_2)_3CH_3$ | H | H | H | Cl |
| $CH_3$ | H | H | $-(CH_2)_3CH_3$ | H | H | $CH_3$ | Cl |
| $CH_3$ | H | H | $-(CH_2)_3CH_3$ | H | H | $-(CH_2)_3CH_3$ | Cl |
| $CH_3$ | H | H | H | H | $CH_3$ | $CH_3$ | Cl |
| $CH_3$ | $-(CH_2)_2CH_3$ | H | H | H | H | H | Cl |
| $CH_3$ | $-(CH_2)_2CH_3$ | H | H | H | H | $CH_3$ | Cl |
| $CH_3$ | $-(CH_2)_2CH_3$ | H | H | H | H | $-(CH_2)_3CH_3$ | Cl |
| $CH_3$ | $-(CH_2)_3CH_3$ | H | H | H | H | H | Cl |
| $CH_3$ | $-(CH_2)_3CH_3$ | H | H | H | H | $CH_3$ | Cl |
| $CH_3$ | $-(CH_2)_3CH_3$ | H | H | H | $CH_3$ | $CH_3$ | Cl |
| $CH_3$ | $-(CH_2)_3CH_3$ | H | $CH_3$ | H | H | H | Cl |
| $CH_3$ | $-CH(CH_3)_2$ | H | H | H | H | H | Cl |
| $CH_3$ | $-CH(CH_3)_2$ | H | H | H | H | $CH_3$ | Cl |
| $CH_3$ | $-CH(CH_3)_2$ | H | H | H | H | $(CH_2)_3CH_3$ | Cl |
| $CH_3$ | $-CH_2-CH(CH_3)_2$ | H | H | H | H | $CH_3$ | Cl |
| $CH_3$ | $-CH_2CH(CH_3)_2$ | H | H | H | H | H | Cl |

| $R^1$ | $R^2$ | $R^3$ | $R^4$ | $R^5$ | $R^6$ | $R^7$ | Y |
|---|---|---|---|---|---|---|---|
| $CH_3$ | $-CH_2CH(CH_3)_2$ | H | H | H | $CH_3$ | $CH_3$ | Cl |
| $CH_3$ | $C_2H_5$ | $C_2H_5$ | H | H | H | H | Cl |
| $CH_3$ | $C_2H_5$ | $C_2H_5$ | H | H | H | $CH_3$ | Cl |
| $CH_3$ | $C_2H_5$ | $C_2H_5$ | H | H | H | $-(CH_2)_3CH_3$ | Cl |
| $CH_3$ | $C_2H_5$ | $C_2H_5$ | H | H | $CH_3$ | $CH_3$ | Cl |
| $CH_3$ | $CH_3$ | $CH_3$ | $CH_3$ | H | H | H | Cl |
| $CH_3$ | $CH_3$ | $CH_3$ | $CH_3$ | H | H | $CH_3$ | Cl |
| $CH_3$ | $CH_3$ | $CH_3$ | $CH_3$ | H | $CH_3$ | $CH_3$ | Cl |
| $CH_3$ | $CH_3$ | H | $CH_3$ | $CH_3$ | H | H | Cl |
| $CH_3$ | $CH_3$ | H | $CH_3$ | $CH_3$ | H | $CH_3$ | Cl |
| $CH_3$ | $CH_3$ | H | $CH_3$ | $CH_3$ | $CH_3$ | $CH_3$ | Cl |
| $CH_3$ | $CH_3$ | $CH_3$ | $CH_3$ | H | $(CH_2)_2CH_3$ | $(CH_2)_2CH_3$ | Cl |
| $CH_3$ | $CH_3$ | $CH_3$ | $CH_3$ | $CH_3$ | H | H | Cl |
| $CH_3$ | $CH_3$ | $CH_3$ | $CH_3$ | $CH_3$ | H | $CH_3$ | Cl |
| $CH_3$ | $CH_3$ | $CH_3$ | $CH_3$ | $CH_3$ | H | $(CH_2)_2CH_3$ | Cl |
| $CH_3$ | $CH_3$ | $CH_3$ | $CH_3$ | $CH_3$ | H | $(CH_2)_5CH_3$ | Cl |
| $CH_3$ | $CH_3$ | $CH_3$ | $CH_3$ | $CH_3$ | $CH_3$ | $CH_3$ | Cl |
| $CH_3$ | $CH_3$ | $CH_3$ | $CH_3$ | $CH_3$ | $(CH_2)_2CH_3$ | $(CH_2)_2CH_3$ | Cl |

Beispiel 1

5-(4-Chlor-3-sulfamoylphenyl)-5-hydroxy-1-methyl-2-oxo-pyrrolidin bzw. 4-(4-Chlor-3-sulfamoylphenyl)-4-oxo-butansäure-methylamid

*(Methode I)*

7,3 g 4-(4-Chlor-3-sulfamoylphenyl)-4-oxobutansäure werden in 50 ml absol. Tetrahydrofuran suspendiert, das Gemisch nach Zugabe von 3,8 ml Triäthylamin auf −5°C abgekühlt und sodann mit 2,6 ml Chlorameisensäureäthylester versetzt. Man rührt etwa 5 bis 10 Minuten bei 0°, versetzt mit 30 ml wäßriger Methylamin-Lösung (40%ig) und läßt langsam auf Raumtemperatur kommen. Das Reaktionsgemisch wird etwa zur Hälfte unter vermindertem Druck eingeengt, mit 2 n HCl unter Kühlung auf pH 6—7 gestellt und der amorphe Niederschlag zur Kristallisation gebracht.

Farblose Kristalle, Schmp. 147—149°C.

Beispiel 2

5-(4-Chlor-3-sulfamoylphenyl)-5-hydroxy-1-methyl-2-oxo-pyrrolidin bzw. 4-(4-Chlor-3-sulfamoylphenyl)-4-oxo-butansäure-methylamid

*(Methode II)*

a) 27 g 4-(4-Chlor-3-sulfamoylphenyl)-4-oxobutansäure werden in 200 ml Methanol gelöst, mit 1 g Acetylchlorid versetzt und bei Raumtemperatur gerührt. Nach 24 Stunden waren nur noch geringe Mengen an Ausgangsmaterial im Dünnschichtchromatogram nachweisbar. Man vertreibt das Lösungsmittel, behandelt den Rückstand mit 200 ml Essigester/200 ml Wasser und rührt. 30 Minuten kräftig, nachdem mit gesättigter Natriumbicarbonat-Lösung ein pH 8 eingestellt wurde. Nach Abtrennung und Trocknung der organischen Phase über Natriumsulfat destilliert man das Lösungsmittel ab und erhält den 4-(4-Chlor-3-sulfamoylphenyl)-4-oxobutansäuremethylester.

Farblose Kristalle, Schmp. 113—115°C.

Analog erhält man beispielsweise aus 4-(4-Chlor-3-sulfamoylphenyl)-4-oxobutansäure und Acetylchlorid in

α) Äthanol den 4-(4-Chlor-3-sulfamoylphenyl)-4-oxobutansäureäthylester (Schmp. 94—95°C)

β) Isopropanol den 4-(4-Chlor-3-sulfamoylphenyl)-4-oxo-butansäureisopropylester (Schmp. 128—130°C).

b) In eine Lösung von 6 g gasförmigen Methylamin in 80 ml Methanol trägt man unter Rührung 8 g 4-(4-Chlor-3-sulfamoylphenyl)-4-oxobutansäuremethylester ein und läßt bei Raumtemperatur stehen. Der Fortgang der Reaktion wird auf dem Dünnschichtchromatogram (Kieselgel, Essigester) verfolgt. Wenn kein Ausgangsmaterial mehr nachweisbar ist (ca. 48 Stunden), vertreibt man das Lösungsmittel unter vermindertem Druck, versetzt den Rückstand mit Wasser, stellt mit 2 n HCL auf pH 6 bis 5 und knetet den amorphen Niederschlag gut unter der wäßrigen Phase durch. Nach dem Stehenlassen über Nacht filtriert man die Kristalle ab.

Schmp. 145—148°C.

Analog erhält man 5-(4-Chlor-3-sulfamoylphenyl)-5-hydroxy-1-methyl-2-oxopyrrolidin beispielsweise auch durch Reaktion von Methylamin mit 4-(4-Chlor-3-sulfamoylphenyl)-4-oxobutansäureäthylester oder mit 4-(4-Chlor-3-sulfamoylphenyl)-4-oxobutansäure-isopropylester.

Beispiel 3

4-(4-Chlor-3-sulfamoylphenyl)-4-oxobutansäure-äthylamid bzw. 1-Äthyl-5-(4-Chlor-3-sulfamoylphenyl)-5-hydroxy-2-oxopyrrolidin

erhält man analog der in Beispiel 2 b) angegebenen Vorschrift durch Umsetzung von 6 g 4-(4-Chlor-3-sulfamoylphenyl)-4-oxobutansäuremethylester mit einer Lösung aus 9 g Äthylamin in 90 ml Methanol.

Farblose Kristalle, Schmp. 164—166°C, Zersetzung ab 182°C.

Beispiel 4

4-(4-Chlor-3-sulfamoylphenyl)-4-oxobutansäure-amid bzw. 5-(4-Chlor-3-sulfamoylphenyl)-5-hydroxy-2-oxopyrrolidin

erhält man analog der in Beispiel 1 angegeben Vorschrift unter Verwendung von 100 ml wäßrigem Ammoniak (25%ig) anstelle der Methylaminlösung.

Farblose Kristalle, Zersetzung ab 173°C.

Beispiel 5

4-(4-Chlor-3-sulfamoylphenyl)-4-oxobutansäure-isopropylamid bzw. 5-(4-Chlor-3-sulfamoylphenyl)-5-hydroxy-1-isopropyl-2-oxopyrrolidin

erhält man analog der in Beispiel 1 angegebenen Vorschrift unter Verwendung einer Lösung von 1,7 g Isopropylamin (anstelle von Methylamin) in 20 ml Tetrahydrofuran. Man rührt das Reaktionsgemisch 20 Stunden bei Raumtemperatur, vertriebt das Lösungsmittel, versetzt mit 100 ml Wasser und extrahiert mit 100 ml Essigester. Nach dem Trocknen über Natriumsulfat destilliert man das Lösungsmittel unter vermindertem Druck ab, rührt den Rückstand 2 Stunden unter Diisopropyläther und filtriert die farblosen Kristalle ab. Schmp. 164°C.

Beispiel 6

4-(4-Chlor-3-sulfamoylphenyl)-4-oxobutansäure-isobutylamid bzw. 5-(4-Chlor-3-sulfamoylphenyl)-5-hydroxy-1-isobutyl-2-oxopyrrolidin

erhält man analog der in Beispiel 1 angegebenen Vorschrift unter Verwendung einer Lösung von 2,2 g Isobutylamin (anstelle von Methylamin) in 20 ml Tetrahydrofuran. Die Aufarbeitung erfolgt analog der in Beispiel 5 angegebenen Vorschrift.

Farblose Kristalle, Schmp. 129°C (aus Chloroform).

Beispiel 7

4-(4-Chlor-3-sulfamoylphenyl)-4-oxobutansäure-(2-methoxypropylamid) bzw. 5-(4-Chlor-3-sulfamoylphenyl)-5-hydroxy-1-(2-methyoxypropyl)-2-oxopyrrolidin

erhält man analog der in Beispiel 1 angegebenen Vorschrift unter Verwendung einer Lösung von 2,6 g 2-Methoxypropylamin (anstelle von Methylamin) in 20 ml Tetrahydrofuran. Die Aufarbeitung erfolgt analog der in Beispiel 5 angegebenen Vorschrift.

Farblose Kristalle aus Isopropanol, Schmp. 127°C.

Beispiel 8

4-(4-Chlor-3-sulfamoylphenyl)-4-oxobutansäure-allylamid bzw. 1-Allyl-5-(4-chlor-3-sulfamoylphenyl)-5-hydroxy-2-oxopyrrolidin

erhält man analog der in Beispiel 1 angegebenen Vorschrift unter Verwendung einer Lösung von 1,6 g Allylamin (anstelle von Methylamin) in 20 ml Tetrahyrofuran und arbeitet analog der in Beispiel 5 angegebenen Vorschrift auf.

Farbloser amorpher Feststoff, Schmp. 86°C unter Zersetzung.

Beispiel 9

4-(4-Chlor-3-sulfamoylphenyl)-4-oxobutansäure-cyclopropylamid bzw. 5-(4-Chlor-3-sulfamoylphenyl)-1-cyclopropyl-5-hydroxy-2-oxopyrrolidin

erhält man analog der in Beispiel 2 b) angegebenen Vorschrift aus 6 g 4-(4-Chlor-3-sulfamoylphenyl)-4-oxo-butansäuremethylester und 6 g Cyclopropylamin und Stehenlassen bei Raumtemperatur über 3 Wochen.

Farblose Kristalle, Zers.p. 128°C.

Beispiel 10

4-(4-Chlor-3-sulfamoylphenyl)-4-oxobutansäure-cyclopentylamid bzw. 5-(4-Chlor-3-sulfamoylphenyl)-1-cyclopentyl-5-hydroxy-2-oxopyrrolidin

erhält man analog der in Beispiel 2 b) angegebenen Vorschrift aus 6 g 4-(4-Chlor-3-sulfamoylphenyl)-4-oxobutansäuremethylester und 6 g Cyclopentylamin und einer Reaktionsdauer von etwa 2 Wochen bei Raumtemperatur.

Farblose Kristalle, Schmp. 153°C (Zers.).

Beispiel 11

5-(4-Brom-3-sulfamoylphenyl)-5-hydroxy-1-methyl-2-oxo-pyrrolidin bzw. 4-(4-Brom-3-sulfamoylphenyl)-4-oxobutansäure-methylamid

a) 10 g 4-(4-Bromphenyl)-4-oxobutansäure werden in 100 ml Salpetersäure ($\delta$ = 1,52) bei —15°C eingetragen, das Reaktionsgemisch 1 1/2 Stunden bei —10°C nachgerührt und sodann unter Rührung auf Eis gegossen. Man filtriert die kristalline 4-(4-Brom-3-nitrophenyl)-4-oxobutansäure ab, wäscht mit Wasser nach.

Farblose bis blassgelbe Kristalle, Schmp. 165°C.

b) Zu einem Gemisch aus 10 g 4-(4-Brom-3-nitrophenyl)-4-oxobutansäure und 6,5 g Eisenpulver tropft man 3,6 ml conc. HCl zu, kocht sodann 4 Stunden am Rückflußkühler und filtriert vom Ungelösten heiß ab. Das Filtrat wird eingedampft, der ölige Rückstand nach Zugabe von Wasser und 5 ml 2 n HCl mit Essigester extrahiert und nach dem Trocknen der organischen Phase über Natriumsulfat das Lösungsmittel abdestilliert. Man erhält farblose kristalline 4-(3-Amino-4-brom-phenyl)-4-oxobutansäure, Schmp. 169—171°C (aus Wasser/Eisessig).

c) Zu einer Suspension von 5 g 4-(3-Amino-4-bromphenyl)-4-oxobutansäure in 50 ml 20%iger Salzsäure tropft man bei 0 bis —5°C eine Lösung von 1,3 g Natriumnitrit in 10 ml Wasser. Das Reaktionsgemisch gießt man zu einer Suspension aus 3,1 g $CuCl_2 \cdot 2\ H_2O$ in 100 ml $SO_2$-gesättiger Eisessiglösung, rührt etwa 30 Minuten und verdünnt sodann mit dem gleichen Volumen Wasser. Man erhält farblose kristalline 4-(4-Brom-3-chlorsulfonylphenyl)-4-oxobutansäure vom Schmp. 183—185°C.

d) 4 g 4-(4-Brom-3-chlor-sulfonylphenyl)-4-oxobutansäure werden in 70 ml wäßrige Ammoniaklösung (25%ig) eingetragen und 6 Stunden bei Raumtemperatur gerührt. Man destilliert sodann etwa 1/4 des Volumens ab, stellt das Gemisch mit Salzsäure auf pH 1 bis 0 und filtriert die kristalline 4-(4-Brom-3-sulfamoylphenyl)-4-oxobutansäure ab, Schmp. 176—178°C (aus Wasser).

e) 1,5 g 4-(4-Brom-3-sulfamoylphenyl)-4-oxobutansäure werden in einem Gemisch aus 0,2 g Acetylchlorid in 30 ml Methanol analog der in Beispiel 2 a) angegebenen Vorschrift in 4-(4-Brom-3-sulfamoylphenyl)-4-oxobutansäure-methylester, Schmp. 110—112°C, übergeführt.

f) 5-(4-Brom-3-sulfamoylphenyl)-5-hydroxy-1-methyl-2-oxo-pyrrolidin erhält man analog der in Beispiel 2 b) angegebenen Vorschrift aus 8 g 4-(4-Brom-3-sulfamoylphenyl)-4-oxobutansäuremethylester und 8 g Methylamin.

Farblose Kristalle, Schmp. 148°C.

Beispiel 12

4-(4-Chlor-3-sulfamoylphenyl)-4-oxobutansäure-benzylamid bzw. 1-Benzyl-5-(4-chlor-3-sulfamoylphenyl)-5-hydroxy-2-oxopyrrolidin

erhält man analog der in Beispiel 1 angegebenen Vorschrift unter Verwendung einer Lösung von 3,2 g Benzylamin (anstelle von Methylamin) in 20 ml Tetrahydrofuran. Die Aufarbeitung erfolgt analog der in Beispiel 5 angegebenen Vorschrift.

Farblose Kristalle, Schmp. 114—116°C.

Beispiel 13

5 - (4 - Chlor - 3 - sulfamoylphenyl) - 5 - hydroxy - 1 - methyl - 2 - oxopyrrolidin bzw. 4 - (4 - chlor - 3-sulfamoylphenyl) - 4 - oxobutansäure - methylamid

erhält man analog der in Beispiel 1 angegebenen Vorschrift unter Verwendung von 3,1 g Methansulfonsäurechlorid anstelle des Chlorameisensäureäthylesters.

Farblose Kristalle. Schmp. 146—148°C. Der Mischschmelzpunkt mit dem Produkt von Beispiel 1 zeigte keine Depression.

Beispiel 14

5-(4-Chlor-3-sulfamoylphenyl)-5-hydroxy-1-methyl-2-oxo-pyrrolidin bzw. 4-(4-Chlor-3-sulfamoylphenyl)-4-oxobutansäure-methylamid

a) Zu einer Mischung aus 7,3 g 4-(4-Chlor-3-sulfamoylphenyl)-4-oxobutansäure, 100 ml Äthanol und 2,5 g Triäthylamin gibt man bei 10°C portionsweise unter Rührung 3,8 g Natriumboronat, rührt weitere 2,5 Stunden bei Raumtemperatur und vertreibt das Lösungsmittel. Der Rückstand wird in wenig Wasser aufgenommen und mit HCl (konz) sauer gestellt. Nach dem Stehen über 2 Tage filtriert man das kristalline 5-(4-Chlor-3-sulfamoylphenyl)-butyrolacton ab.

Schmp. 156—157°C (aus Diisopropyläther).

b) 2,4 g 5-(4-Chlor-3-sulfamoylphenyl)-butyrolacton werden in 60 ml Methanol mit 1 g Methylamin 15 Stunden bei Raumtemperatur gerührt, sodann 1 Stunde im Autoklaven auf 100°C erhitzt, und das Lösungsmittel unter vermindertem Druck abdestilliert. Der ölige Rückstand, bestehend aus 4-(4-Chlor-3-sulfamoylphenyl)-4-hydroxybutansäure-methylamid wird in 100 ml Acetonitril gelöst und nach Zugabe von 20 g aktive Mangan (IV) oxid 3 Stunden bei Raumtemperatur gerührt und filtriert. Der anorganische Rückstand wird 1 × mit Aceton gewaschen, die vereinigten Phasen eingeengt.

Farblose Kristalle, Fp. 146—148°C.

Beispiel 15

5-(4-Chlor-3-methylsulfamoylphenyl)-5-hydroxy-1-methyl-2-oxo-pyrrolidin bzw. 4-(4-Chlor-3-methylsulfamoyl)-4-oxobutansäure-methylamid

a) 15,5 g 4-(4-Chlor-3-chlorsulfamoylphenyl)-4-oxobutansäure werden in eine Lösung aus 10 g gasförmigen Methylamin in 100 ml Methanol portionsweise unter Eiskühlung und Rührung eingetragen und über 18 Stunden bei Raumtemperatur gerührt. Man verjagt das Lösungsmittel, versetzt den Rückstand mit 300 ml Wasser, stellt mit konz. HCl sauer und filtriert die kristalline 4-(4-Chlor-3-methylsulfamoylphenyl)-4-oxobutansäure vom Schmp. 144°C ab.

b) Eine Mischung von 12 g 4-(4-Chlor-3-methylsulfamoylphenyl)-4-oxobutansäure und 1,2 g Acetylchlorid in 120 ml Methanol wird 48 Stunden analog der in Beispiel 2a) angegebenen Vorschrift umgesetzt und aufgearbeitet. Man erhält 4-(4-Chlor-3-methylsulfamoylphenyl)-4-oxobutansäuremethylester vom Schmp. 112—114°C (aus Äthanol).

c) 7,9 g 4-(4-Chlor-3-methylsulfamoylphenyl)-4-oxobutansäuremethylester werden mit 6,9 g Methylamin in 100 ml Methanol umgesetzt und analog der in Beispiel 2 b) angegebenen Vorschrift aufgearbeitet. Man erhält 5-(4-Chlor-3-methylsulfamoylphenyl)-5-hydroxy-1-methyl-2-oxopyrrolidin vom Schmp. 147—150°C.

Beispiel 16

5-(4-Chlor-3-propylsulfamoylphenyl)-5-hydroxy-1-methyl-2-oxopyrrolidin bzw. 4-(4-Chlor-3-propylsulfamoylphenyl)-4-oxobutansäure-methylamid

a) 4-(4-Chlor-3-propylsulfamoylphenyl)-4-oxobutansäure erhält man analog der in Beispiel 15 a) angegebenen Vorschrift bei Verwendung von 9 g n-Propylamin anstelle des Methylamins.

Farblose Kristalle, Schmp. 106°C.

b) 4-(4-Chlor-3-propylsulfamoylphenyl)-4-oxobutansäuremethylester erhält man analog der in Beispiel 2 a) angegebenen Vorschrift.

Schmp. 93—94°C.

c) 5-(4-Chlor-3-propylsulfamoylphenyl)-5-hydroxy-1-methyl-2-oxopyrrolidin erhält man analog der in Beispiel 2 b) angegebenen Vorschrift aus 10,1 g 4-(4-Chlor-3-propylsulfamoylphenyl)-4-oxobutansäuremethylester und 7 g Methylamin.

Schmp. 167—169°C.

Beispiel 17

4-(4-Chlor-3-methylsulfamoylphenyl)-4-oxobutansäureamid bzw. 5-(4-Chlor-3-methylsulfamoylphenyl)-5-hydroxy-2-oxopyrrolidin

erhält man analog der in Beispiel 1 angegebenen Vorschrift unter Verwendung von 7,5 g 4-(4-Chlor-3-methylsulfamoylphenyl)-4-oxobutansäure, 3,8 ml Triäthylamin, 2,6 ml Chlorameisensäureäthylester und 150 ml 25%ige wäßrige Ammoniaklösung in Tetrahydrofuran als Lösungsmittel.

Schmp. 108—110°C.

Beispiel 18

5-(4-Chlor-3-sek.butylsulfamoylphenyl)-5-hydroxy-1-methyl-2-oxopyrrolidin bzw. 4-(4-Chlor-3-sek.-butylsulfamoylphenyl)-4-oxobutansäure-methylamid

a) 4-(4-Chlor-3-sek.butylsulfamoylphenyl)-4-oxobutansäure erhält man analog der in Beispiel 15 a) angegebenen Vorschrift bei Verwendung von 13 g sek. Butylamin anstelle von Methylamin.

Schmp. 133°C.

b) 5-(4-Chlor-3-sek.butylsulfamoylphenyl)-5-hydroxy-1-methyl-2-oxopyrrolidin erhält man analog der in Beispiel 1 angegebenen Vorschrift bei Verwendung von 4,2 g 4-(4-Chlor-3-sek.butylsulfamoylphenyl)-4-oxobutansäure, 1,7 ml Triäthylamin, 1,2 ml Chlorameisensäureäthylester und 20 ml 40%ige wäßrige Methylaminlösung in Tetrahydrofuran als Lösungsmittel.

Farblose Kristalle vom Schmp. 138°C.

Beispiel 19

5-(4-Chlor-3-n-hexylsulfamoylphenyl)-5-hydroxy-1-methyl-2-oxopyrrolidin bzw. 4-(4-Chlor-3-n-hexylsulfamoylphenyl)-4-oxobutansäure-N-methylamid

erhält man analog der in Beispiel 1 angegebenen Vorschrift aus 7,5 g 4-(4-Chlor-3-n-hexylsulfamoylphenyl)-4-oxobutansäure und 100 ml 40%iger wäßriger Methylaminlösung unter Verwendung von 3,2 Triäthylamin und 2,2 ml Chlorameisensäureäthylester.

Farblose Kristalle, Schmp. 114—117°C.

Die erforderliche 4-(4-Chlor-3-n-hexylsulfamoylphenyl)-4-oxobutansäure erhält man analog der in Beispiel 15 a) angegebenen Vorschrift unter Verwendung von 7,5 g n-Hexylamin.

Farblose Kristalle, Schmp. 115—118°C.

Beispiel 20

5-(4-Chlor-3-isobutylsulfamoylphenyl)-5-hydroxy-1-methyl-2-oxopyrrolidin bzw. 4-(4-Chlor-3-isobutylsulfamoylphenyl)-4-oxobutansäure-N-Methylamid

a) 4-(4-Chlor-3-isobutylsulfamoylphenyl)-4-oxobutansäure erhält man analog der in Beispiel 15 a) angegebenen Vorschrift aus 8 g Isobutylamin und 9,3 g 4-(4-Chlor-3-chlorsulfonylphenyl)-4-oxobutansäure.

Farblose Kristalle, Schmp. 128—130°C.

b) 5-(4-Chlor-3-isobutylsulfamoylphenyl)-5-hydroxy-1-methyl-2-oxopyrrolidin erhält man analog der in Beispiel 1 angegebenen Vorschrift aus 4-(4-Chlor-3-isobutylsulfamoylphenyl)-4-oxobutansäure und 100 ml 40%ige wäßrige Methylaminlösung unter Verwendung von 3,2 ml Triäthylamin und 2,2 ml Chlorameisensäureäthylester.

Farblose Kristalle, Schmp. 144—147°C.

Beispiel 21

5-(3-Allylsulfamoyl-4-chlorphenyl)-5-hydroxy-1-methyl-2-oxopyrrolidin bzw. 4-(3-Allylsulfamoyl-4-chlorphenyl)-4-oxobutansäure-N-methylamid

a) 4-(3-Allylsulfamoyl-4-chlorphenyl)-4-oxobutansäure erhält man analog der in Beispiel 15 a) angegebenen Vorschrift aus 9,3 g 4-(4-Chlor-3-chlorsulfonylphenyl)-4-oxobutansäure und 6 g Allylamin.

Farblose Kristalle, Schmp. 107—109°C.

b) 5-(3-Allylsulfamoyl-4-chlorphenyl)-5-hydroxy-1-methyl-2-oxopyrrolidin erhält man analog der in Beispiel 1 — angegebenen Vorschrift aus 6,6 g 4-(3-Allylsulfamoyl-4-chlorphenyl)-4-oxobutansäure und 100 ml 40%ige wäßrige Methylaminlösung unter Verwendung von 3,2 g Triäthylamin und 2,2 ml Chlorameisensäureäthylester.

Farblose Kristalle, Schmp. 133—135°C.

Beispiel 22

5-(4-Chlor-3-cyclopentylsulfamoylphenyl)-5-hydroxy-1-methyl-2-oxopyrrolidin bzw. 4-(4-Chlor-3-cyclopentylsulfamoylphenyl)-4-oxobutansäure-N-methylamid

a) 4-(4-Chlor-3-cyclopentylsulfamoylphenyl)-4-oxobutansäure erhält man analog der in Beispiel

15 a) angegebenen Vorschrift aus 9,3 g 4-(4-Chlor-3-chlorsulfonylphenyl)-4-oxobutansäure und 9 g Cyclopentylamin.

Farblose Kristalle, Schmp. 144—146°C.

b) 5-(4-Chlor-3-cyclopentylsulfamoylphenyl)-5-hydroxy-1-methyl-2-oxopyrrolidin erhält man analog der in Beispiel 1 angegebenen Vorschrift aus 6,5 g 4-(4-Chlor-3-cyclopentyl-sulfamoylphenyl)-4-oxobutansäure und 40 ml 40%iger wäßriger Methylaminlösung unter Verwendung von 2,6 ml Triäthylamin und 1,8 ml Chlorameisensäureäthylester.

Farblose Kristalle, Schmp. 136—139°C.

Beispiel 23

5-(4-Chlor-3-cyclohexylsulfamoylphenyl)-5-hydroxy-1-methyl-2-oxopyrrolidin bzw. 4-(4-Chlor-3-cyclohexylsulfamoylphenyl)-4-oxobutansäure-N-methylamid

a) 4-(4-Chlor-3-cyclohexylsulfamoylphenyl)-4-oxobutansäure erhält man analog der in Beispiel 15 a) angegebenen Vorschrift aus 9,3 g 4-(4-Chlor-3-chlorsulfonylphenyl)-4-oxobutansäure und 9 g Cyclohexylamin. Schmp. 133—134°C.

b) 5-(4-Chlor-3-cyclohexylsulfamoylphenyl)-5-hydroxy-1-methyl-2-oxopyrrolidin erhält man analog der in Beispiel 1 angegebenen Vorschrift aus 6 g 4-(4-Chlor-3-cyclohexylsulfamoylphenyl)-4-oxobutansäure und 40 ml 40%iger wäßriger Methylaminlösung unter Verwendung von 1,8 ml Chlorameisensäureäthylester und 2,5 ml Triäthylamin und arbeitet nach der in Beispiel 5 angegebenen Vorschrift auf.

Farblose Kristalle, Schmp. 148—150°C.

Beispiel 24

5-(4-Chlor-3-dipropylsulfamoylphenyl)-5-hydroxy-1-methyl-2-oxopyrrolidin bzw. 4-(4-Chlor-3-dipropylsulfamoylphenyl)-4-oxobutansäure-N-methylamid

a) 4-(4-Chlor-3-dipropylsulfamoylphenyl)-4-oxobutansäure erhält man analog der in Beispiel 15 a) angegebenen Vorschrift aus 9,3 g 4-(4-Chlor-3-chlorsulfonylphenyl)-4-oxobutansäure und 10 g Dipropylamin.

Farblose Kristalle, Schmp. 100—102°C.

b) 5-(4-Chlor-3-dipropylsulfamoylphenyl)-5-hydroxy-1-methyl-2-oxopyrrolidin erhält man analog der in Beispiel 1 angegebenen Vorschrift aus 7,5 g 4-(4-Chlor-3-dipropylsulfamoylphenyl)-oxobutansäure und 100 ml 40%ige wäßrige Methylaminlösung unter Verwendung von Triäthylamin und Chlorameisensäureäthylester.

Schmp. 110—114°C.

Beispiel 25

5-(4-Chlor-3-dimethylsulfamoylphenyl)-5-hydroxy-1-methyl-2-oxopyrrolidin bzw. 4-(4-Chlor-3-dimethylsulfamoylphenyl-4-oxobutansäure-N-methylamid

a) 4-(4-Chlor-3-dimethylsulfamoylphenyl)-4-oxobutansäure erhält man analog der in Beispiel 15 a) angegebenen Vorschrift aus 15,5 g 4-(4-Chlor-3-chlorsulfonylphenyl)-4-oxobutansäure und einer Lösung von 10 g Dimethylamin in 150 ml Methanol.

Farblose Kristalle, Schmp. 114—116°C.

b) 4-(4-Chlor-3-dimethylsulfamoylphenyl)-4-oxobutansäuremethylester erhält man analog der in Beispiel 2 a) beschriebenen Vorschrift aus 4-(4-Chlor-3-dimethylsulfamoylphenyl)-4-oxobutansäure und Methanol in Gegenwart von Acetylchlorid.

Farblose Kristalle, Schmp. 55—56°C.

c) 5-(4-Chlor-3-dimethylsulfamoylphenyl)-5-hydroxy-1-methyl-2-oxopyrrolidin erhält man analog der in Beispiel 2 b) angegebenen Vorschrift aus 8,8 g 4-(4-Chlor-3-dimethylsulfamoylphenyl)-4-oxobutansäuremethylester und einer Lösung von 6 g Methylamin in 100 ml Methanol.

Farblose Kristalle, Schmp. 128—131°C.

Beispiel 26

5-[4-Chlor-3-(2-phenyläthylsulfamoyl)-phenyl]-5-hydroxy-1-methyl-2-oxopyrrolidin bzw. 4-[4-Chlor-3-(2-phenyl-äthylsulfamoylphenyl)-4-oxobutansäure-N-methylamid

a) 4-[4-Chlor-3-(2-phenyläthylsulfamoyl)-phenyl]-4-oxo-butansäure erhält man durch Zugabe von 10 g 4-(4-Chlor-3-chlorsulfonylphenyl)-4-oxobutansäure zu einer Lösung aus 5,45 g 2-Phenyläthylamin und 8,11 g Triäthylamin unter Eiskühlung und Rührung bei Raumtemperatur über 12 Stunden, ansäuern mit HCl und Kristallisation des amorphen Niederschlages unter frischem Wasser über 30 bis 40 Stunden.

Man dekantiert ab, versetzt den Rückstand mit wenig Methanol und filtriert die Kristalle nach Rühren über eine Stunde ab.

Schmp. 147—149°C.

b) 5-[4-Chlor-3-(2-phenyläthylsulfamoyl)-phenyl]-5-hydroxy-1-methyl-2-oxopyrrolidin erhält man analog der in Beispiel 1 angegebenen Vorschrift aus 4,6 g 4-[4-Chlor-3-(2-phenyläthylsulfamoyl)-phenyl]-4-oxo-butansäure, 1,6 ml Triäthylamin und 1,2 ml Chlorameisensäureäthylester.

Schmp. 149—153°C.

Beispiel 27

5-(4-Chlor-3-N-benzyl-N-methylsulfamoylphenyl)-5-hydroxy-1-methyl-2-oxopyrrolidin bzw. 4-(4-Chlor-3-N-benzyl-N-methylsulfamoylphenyl)-4-oxobutansäure-N-methylamid

a) 4-(4-Chlor-3-N-benzyl-N-methylsulfamoylphenyl)-4-oxobutansäure erhält man analog der in Beispiel 26 a) angegebenen Vorschrift aus 10 g 4-(4-Chlor-3-chlorsulfonylphenyl)-4-oxobutansäure, 5.4 g N-Methyl-N-benzylamin und 8,1 g Triäthylamin in Methanol.

Schmp. 73°C.

b) 5-[4-Chlor-3-(2,4-dimethoxybenzylsulfamoyl)-phenyl]-5-hydroxy-1-methyl-2-oxopyrrolidin erhält man analog der in Beispiel 1 angegebenen Vorschrift aus 7 g 4-(4-Chlor-3-N-benzyl-N-methylsulfamoylphenyl)-4-oxobutansäure und 60 ml 40%iger wäßriger Methylaminlösung unter Verwendung von 2,4 ml Triäthylamin und 1,8 ml Chlorameisensäureäthylester.

Farblose Kristalle, Schmp. 116—119°C.

Beispiel 28

5-[4-Chlor-3-(2,4-dimethoxybenzylsulfamoyl)-phenyl]-5-hydroxy-1-methyl-2-oxopyrrolidin bzw. 4-[4-Chlor-3-(2,4-dimethoxybenzylsulfamoyl)-phenyl]-4-oxobutansäure-N-methylamid

a) 4-[4-Chlor-3-(2,4-dimethoxybenzylsulfamoyl)-phenyl]-4-oxobutansäure erhält man analog Vorschrift 26 a) aus 9,4 g 4-(4-Chlor-3-chlorsulfonylphenyl)-4-oxobutansäure, 5,4 g 2,4-Dimethoxybenzylamin und 8,5 g Triäthylamin.

Schmp. 167°C.

b) 5-[4-Chlor-3-(2,4-dimethoxybenzylsulfamoyl)-phenyl]-5-hydroxy-1-methyl-2-oxopyrrolidin erhält man analog der in Beispiel 1 angegebenen Vorschrift aus 6,6 g 4-[4-Chlor-3-(2,4-dimethoxybenzylsulfamoyl)-phenyl]-4-oxobutansäure und 80 ml wäßrige 40%ige Methylaminlösung unter Verwendung von 1,7 ml Chlorameisensäureäthylester und 2,4 ml Triäthylamin.

Farblose Kristalle, Schmp. 164—166°C.

Beispiel 29

5-[4-Chlor-3-(2-methoxy-1-propylsulfamoyl)-phenyl]-5-hydroxy-1-methyl-2-oxopyrrolidin bzw. 4-[4-Chlor-3-(2-methoxy-1-propylsulfamoyl)-phenyl]-4-oxobutansäure-N-methylamid

a) 4-[4-Chlor-3-(2-methoxy-1-propylsulfamoyl)-phenyl]-4-oxobutansäure erhält man analog der in Beispiel 15a) angegebenen Vorschrift aus 10 g 4-(4-Chlor-3-chlor-sulfonylphenyl)-4-oxobutansäure und 9,7 g 2-Methoxy-1-propylamin.

Schmp. 121°C.

b) 5-[4-Chlor-3-(2-methoxy-1-propylsulfamoyl)-phenyl]-5-hydroxy-1-methyl-2-oxopyrrolidin erhält man analog der in Beispiel 1 angegebenen Vorschrift aus 8 g 4-[4-Chlor-3-(2-methoxy-1-propylsulfamoyl)-phenyl]-4-oxo-butansäure, 3 ml Triäthylamin und 2,2 ml Chlorameisensäureäthylester.

Schmp. 124—126°C.

Beispiel 30

5-[3-(2,2-Diäthoxyäthylsulfamoyl)-4-chlorphenyl]-5-hydroxy-1-methyl-2-oxopyrrolidin bzw. 4-[3-(2,2-Diäthoxyäthylsulfamoyl)-4-chlorphenyl]-4-oxobutansäure-N-methylamid

a) 4-[3-(2,2-Diäthoxyäthylsulfamoyl)-4-chlorphenyl]-4-oxobutansäure erhält man analog der in Beispiel 15 a) angegebenen Vorschrift aus 13,4 g Aminoacetaldehyd-diäthylacetal und 9,4 g 4-(4-Chlor-3-chlorsulfonylphenyl)-4-oxobutansäure.

Schmp. 142°C.

b) 5-[3-(2,2-Diäthoxyäthylsulfamoyl)-4-chlorphenyl]-5-hydroxy-1-methyl-2-oxopyrrolidin erhält man analog der in Beispiel 1 angegebenen Vorschrift aus 8,1 g 4-[3-(2,2-Diäthoxyäthylsulfamoyl)-4-chlorphenyl]-4-oxobutansäure und 100 ml 40%ige wäßrige Methylaminlösung unter Verwendung von 3,2 ml Triäthylamin und 2,2 ml Chlorameisensäureäthylester.

Farblose Kristalle, Schmp. 135—138°C.

Beispiel 31

5-[4-Chlor-3-(2-chlorbenzylsulfamoyl)-phenyl]-5-hydroxy-1-methyl-2-oxopyrrolidin bzw. 4-[4-Chlor-3-(2-chlorbenzylsulfamoyl)-phenyl]-4-oxobutansäure-N-methylamid

a) 4-[4-Chlor-3-(2-chlorbenzylsulfamoyl)-phenyl]-4-oxo-butansäure erhält man analog der in Beispiel 26 a) angegebenen Vorschrift aus 9,4 g 4-(4-Chlor-3-chlorsulfonylphenyl)-4-oxobutansäure, 5 g 2-Chlorbenzylamin und 8,5 g Triäthylamin.

Schmp. 158°C.

b) 5-[4-Chlor-3-(2-chlorbenzylsulfamoyl)-phenyl]-5-hydroxy-1-methyl-2-oxopyrrolidin erhält man analog der in Beispiel 1 angegebenen Vorschrift aus 6,3 g 4-[4-Chlor-3-(2-chlorbenzylsulfamoyl)-phenyl]-4-oxobutansäure und 75 ml wäßrige 40%ige Methylaminlösung unter Verwendung von 2,4 ml Triäthylamin und 1,7 ml Chlorameisensäureäthylester.

Schmp. 144—145°C.

Beispiel 32

5-(4-Chlor-3-sulfamoylphenyl)-5-hydroxy-1-methyl-2-oxo-pyrrolidin bzw. 4-(4-Chlor-3-sulfamoylphenyl)-4-oxobutansäure-methylamid

a) 4-(4-Chlor-3-sulfamoylphenyl)-4-oxobutansäure-N-benzyl-N-methylamid erhält man analog der in Beispiel 1 angegebenen Vorschrift aus 7,3 g 4-(4-Chlor-3-sulfamoylphenyl)-3-oxobutansäure und 3,2 g N-Benzyl-N-methylamin unter Verwendung von 3,8 ml Triäthylamin und 2,6 ml Chlorameisensäureäthylester.

Farblose Kristalle aus Essigester, Schmp. 162°C.

b) 2 g 4-(4-Chlor-3-sulfamoylphenyl)-4-oxobutansäure-N-benzyl-N-methylamid werden in Gegenwart von 0,5 g Palladium-Mohr in 40 ml Methanol in einer Schüttelente bis zur Aufnahme der theoretischen Wasserstoffmenge hydriert, vom Katalysator abfiltriert, eingeengt und der Rückstand analog Vorschrift 1 zum 5-(4-Chlor-3-sulfamoylphenyl)-5-hydroxy-1-methyl-2-oxopyrrolidin aufgearbeitet.

Schmp. 147—149°C.

Beispiel 33

5 - (4 - Chlor - 3 - dimethylsulfamoylphenyl) - 5 - hydroxy - 1 - methyl - 2 - oxopyrrolidin bzw. 4 - (4-Chlor - 3 - dimethylsulfamoylphenyl) - 4 - oxobutansäure - methylamid

Zu einer intensiv gerührten Lösung von 0,05 Mol Butyllithium in 150 ml absol. Tetrahydrofuran tropft man unter Ausschluß von Sauerstoff und Luftfeuchtigkeit langsam bei —45°C 14,9 g 5-Brom-2-Chlorbenzolsulfonsäure-dimethylamid in 100 ml absol. Tetrahydrofuran und rührt weitere 20 bis 30 Min. bei —40°C. Zu der so erhaltenen Lösung von 4-Chlor-3-dimethylsulfamoylphenyl-lithium tropft man im Verlauf von etwa 45 Min. eine Lösung von 5 g N-Methysuccinimid in etwa 150 ml absol. Tetrahydrofuran. Das Reaktionsgemisch wird anschließend über Nacht bei Raumtemperatur gerührt und 1 bis 2 Stunden am Rückflußkühler gekocht. Nach dem Abkühlen zersetzt man unter Kühlung durch Zugabe von 25 ml gesättigter wäßriger Ammoniumchlorid-Lösung, filtriert den Niederschlag ab und trocknet das Filtrat kurz über Natriumsulfat. Man vertreibt das Lösungsmittel und bringt den Rückstand unter 70 bis 100 ml Wasser zur Kristallisation.

Farblose Kristalle, Schmp. 145—147°C.

Beispiel 34

5-[4-Chlor-3-(1-pyrrolidinylsulfonyl)-phenyl]-5-hydroxy-1-methyl-2-oxopyrrolidin bzw. 4-[4-Chlor-3-(1-pyrrolidinylsulfonyl)-phenyl]-4-oxobutansäure-N-methylamid

erhält man analog der in Beispiel 1 angegebenen Vorschrift aus 7 g 4-[4-Chlor-3-(1-pyrrolidinylsulfonyl)phenyl]-4-oxobutansäure und 40 ml 40%ige wäßrige Methylaminlösung unter Verwendung von 70 ml Tetrahydrofuran, 2,7 ml Triäthylamin und 2,1 ml Chlorameisensäureäthylester.

Farblose Kristalle, Schmp. 182°C (aus Isopropanol).

Die verwendete 4-[4-Chlor-3-(1-pyrrolidinylsulfonyl)-phenyl]-4-oxobutansäure erhält man analog der in Beispiel 26 a) angegebenen Vorschrift aus 15,5 g 4-(4-Chlor-3-chlorsulfonylphenyl)-4-oxobutansäure und 10,8 g Pyrrolidin.

Farblose Kristalle, Schmp. 142—145°C.

Beispiel 35

5-[4-Chlor-3-(4-chlorbenzylsulfamoyl)-phenyl]-5-hydroxy-methyl-2-oxopyrrolidin bzw. 4-[4-Chlor-3-(4-chlorbenzylsulfamoyl)-phenyl]-4-oxobutansäure-N-methylamid

erhält man analog der in Beispiel 1 angegebenen Vorschrift aus 8,4 g 4-[4-Chlor-3-(4-chlorbenzyl-sulfamoyl)-phenyl]-4-oxobutansäure und 100 ml 40%iger wäßriger Methylaminlösung unter Verwendung von 100 ml Tetrahydrofuran, 3,2 ml Triäthylamin und 2,2 ml Chlorameisensäureäthylester.

Farblose Kristalle aus Isopropanol, Schmp. 179°C.

Die verwendete 4-[4-Chlor-3-(4-chlorbenzylsulfamoyl)-phenyl]-4-oxobutansäure erhält man analog der in Beispiel 26 a) angegebenen Vorschrift aus 9,4 g 4-(4-Chlor-3-chlorsulfonylphenyl)-4-oxobutansäure, 5 g 4-Chlorbenzylamin und 8,5 g Triäthylamin in 150 ml Methanol.

Farblose Kristalle, Schmp. 158°C.

Beispiel 36

5-(4-Chlor-3-cyclopropylsulfamoylphenyl)-5-hydroxy-1-methyl-2-oxopyrrolidin bzw. 4-(4-Chlor-3-cyclopropylsulfamoylphenyl)-4-oxobutansäure-N-methylamid

erhält man analog der in Beispiel 1 angegebenen Vorschrift aus 8 g 4-(4-Chlor-3-cyclopropylsulfamoylphenyl)-4-oxobutansäure und 50 ml 40%iger Methylaminlösung (wäßrig) unter Verwendung von 3,3 ml Triäthylamin und 2,4 ml Chlorameisensäureäthylester in 80 ml Tetrahydrofuran.

Farblose Kristalle aus Isopropanol, Schmp. 184—185°C.

Die verwendete 4-(4-Chlor-3-cyclopropylsulfamoylphenyl)-4-oxobutansäure erhält man analog

der in Beispiel 26 a) angegebenen Vorschrift aus 10 g 4-(4-Chlor-3-chlorsulfonylphenyl)-4-oxobutansäure, 2,5 g Cyclopropylamin, 8 g Triäthylamin und 150 ml Methanol.

Farblose Kristalle, Schmp. 135°C.

Beispiel 37

5-(4-Chlor-3-cyclooctylsulfamoylphenyl)-5-hydroxy-1-methyl-2-oxopyrrolidin bzw. 4-(4-Chlor-3-cyclododecylsulfamoylphenyl)-4'-oxobutansäure-N-methylamid

erhalt man analog der in Beispiel 1 angegebenen Vorschrift aus 4,8 g 4-(4-Chlor-3-cyclooctylsulfamoylphenyl)-4-oxobutansäure, 40 ml 40%ige wäßrige Methylaminlösung unter Verwendung von 1,2 ml Chlorameisensäureäthylester und 1,6 ml Triäthylamin in 50 ml absol. Tetrahydrofuran.

Farbloser Feststoff, Schmp. 112°C.

Die verwendete 4-(4-Chlor-3-cyclooctylsulfamoylphenyl)-4-oxobutansäure erhält man analog der in Beispiel 26 a) angegebenen Vorschrift aus 4-(4-Chlor-3-chlorsulfonylphenyl)-4-oxobutansäure, Cyclooctylamin und Triäthylamin.

Farblose Kristalle, Schmp. 127°C.

Beispiel 38

5-(4-Chlor-3-cyclododecylsulfamoylphenyl)-5-hydroxy-1-methyl-2-oxopyrrolidin bzw. 4-(4-Chlor-3-cyclododecylsulfamoylphenyl)-4-oxobutansäure-N-methylamid

erhält man analog der in Beispiel 1 angegebenen Vorschrift aus 6.4 g 4-(4-Chlor-3-cyclododecylsulfamoylphenyl)-4-oxobutansäure, 1,9 ml Triäthylamin, 1,61 ml Chlorameisensäureäthylester und 50 ml einer 40%igen wäßrigen Methylaminlösung.

Farblose Kristalle aus Isopropanol, Schmp. 192°C.

Die verwendete 4-(4-Chlor-3-cyclododecylsulfamoyl-phenyl)-4-oxobutansäure erhält man analog der in Beispiel 26 a) angegebenen Vorschrift aus 4-(4-Chlor-3-chlorsulfonyl-phenyl)-4-oxobutansäure, Cyclododecylamin und Triäthylamin.

Farblose Kristalle aus Wasser/Eisessig, Schmp. 140°C.

Beispiel 39

5-[4-Chlor-3-(4-methylbenzylsulfamoyl)-phenyl]-5-hydroxy-1-methyl-2-oxopyrrolidin bzw. 4-[4-chlor-3-(4-methylbenzylsulfamoyl)-phenyl]-4-oxobutansäure-N-methylamid

erhält man analog der in Beispiel 1 angegebenen Vorschrift aus 4,3 g 4-[4-Chlor-3-(4-Methylbenzylsulfamoyl)phenyl]-4-oxobutansäure und 50 ml 40%ige wäßrige Methylaminlösung unter Verwendung von 50 ml Tetrahydrofuran, 1,6 g Triäthylamin und 1,1 ml Chlorameisensäureäthylester.

Farblose Kristalle.

Die verwendete 4-[4-Chlor-3-(4-methylbenzylsulfamoyl)-phenyl]-4-oxobutansäure erhält man analog der in Beispiel 26 a) angegebenen Vorschrift aus 9,4 g 4-(4-Chlor-3-chlorsulfonylphenyl)-4-oxobutansäure, 4,3 g 4-Methylbenzylamin und 8,5 g Triäthylamin in 150 ml Methanol.

Schmp. 129—131°C.

Beispiel 40

5-(3-Benzylsulfamoyl-4-chlorphenyl)-5-hydroxy-1-methyl-2-oxopyrrolidin bzw. 4-(3-Benzylsulfamoyl-4-chlorphenyl)-4-oxobutansäure-N-methylamid

erhält man analog der in Beispiel 1 angegebenen Vorschrift aus 9,5 g 4-(3-Benzylsulfamoyl-4-chlorphenyl)-4-oxobutansäure und 100 ml 40%iger wäßriger Methylaminlösung unter Verwendung von 100 ml Tetrahydrofuran, 4 ml Triäthylamin und 2,75 ml Chlorameisensäureäthylester.

Farblose Kristalle, Schmp. 134—136°C (aus Isopropanol).

Die verwendete 4-(3-Benzylsulfamoyl-4-chlorphenyl)-4-oxobutansäure erhält man analog der in Beispiel 26 a) angegebenen Vorschrift aus 15,5 g 4-(4-Chlor-3-chlorsulfonylphenyl)-4-oxobutansäure und 15 g Benzylamin in Methanol.

Farblose Kristalle, Schmp. 153°C.

Beispiel 41

5-[4-Chlor-3-(1-pyrrolidinylsulfonyl)-phenyl]-5-hydroxy-1-methyl-2-oxopyrrolidin bzw. 4-[4-Chlor-3-(1-pyrrolidinylsulfonyl)-phenyl]-4-oxobutansäure-N-methylamid

a) 3,9 g 4-[4-Chlor-3-(1-pyrrolidinylsulfonyl)-phenyl]-4-oxobutansäure werden in 50 ml Acetanhydrid 1 Stunde am Rückflußkühler gekocht, vertreibt das Lösungsmittel unter vermindertem Druck und bringt den Rückstand unter Diisopropyläther zur Kristallisation. Das 5-[4-Chlor-3-(1-pyrrolidinylsulfonyl)-phenyl]-2,3-dihydro-2-oxofuran wird in Form hellrot gefärbter Kristalle vom Schmp. 108—111°C erhalten.

b) 2,8 g 5-[4-Chlor-3-(1-pyrrolidinylsulfonyl)-phenyl]-4-oxobutansäure werden in 40 ml 40%iger wäßriger Methylaminlösung 16 Stunden bei Raumtemperatur gerührt, das Lösungsmittel unter vermindertem Druck abdestilliert und der Rückstand mit 15—20 ml Wasser versetzt. Man stellt mit 2 n HCl auf pH 7 und filtriert die Kristalle ab.

Farblose Kristalle, Schmp. 184°C.

Beispiel 42

5-(4-Chlor-3-n-octylsulfamoylphenyl)-5-hydroxy-1-methyl-2-oxopyrrolidin bzw. 4-(4-Chlor-3-n-octylsulfamoylphenyl)-4-oxobutansäure-N-methylamid

erhält man analog der in Beispiel 1 angegebenen Vorschrift aus 8 g 4-(4-Chlor-3-n-octylsulfamoylphenyl)-4-oxobutansäure unter Verwendung von 2,2 ml Chlorameisensäureäthylester, 3,2 ml Triäthylamin, 100 ml 40%iger wäßriger Methylaminlösung und 100 ml Tetrahydrofuran. Langsame Abscheidung von Kristallen in Methanol,

Schmp. 126—128°C.

Die verwendete 4-(4-Chlor-3-n-octylsulfamoylphenyl)-4-oxobutansäure erhält man analog Beispiel 15 a) aus 9,4 g 4-(4-Chlor-3-chlorsulfonylphenyl)-4-oxobutansäure, 13 g n-Octylamin und 150 ml Methanol. Schmp. 83—85°C.

Beispiel 43

5-(4-Chlor-3-cyclohexylmethylsulfamoylphenyl)-5-hydroxy-1-methyl-2-oxopyrrolidin bzw. 4-(4-Chlor-3-cyclohexylmethylsulfamoyl-phenyl)-4-oxobutansäure-N-methylamid

erhält man analog der in Beispiel 1 angegebenen Vorschrift aus 6,9 g 4-(4-chlor-3-cyclohexylmethylsulfamoyl-phenyl)-4-oxobutansäure in 70 ml abs. Tetrahydrofuran unter Verwendung von 2,4 ml Triäthylamin, 1,8 ml Chlorameisensäureäthylester und 80 ml 40%iger wäßriger Methylaminlösung. Nach der Extraktion der wäßrigen Phase mit Essigester und Trocknen über $Na_2SO_4$ engt man die organische Phase ein und bringt den Rückstand unter Diisopropyläther zur Kristallisation.

Farblose Kristalle. Schmp. 138—140°C.

Die verwendete 4-(4-Chlor-3-cyclohexylmethylsulfamoylphenyl)-4-oxobutansäure erhält man analog der in Beispiel 26 a) angegebenen Vorschrift aus 10 g 4-(4-Chlor-3-chlorsulfonylphenyl)-4-oxobutansäure, 5,1 g Aminomethylcyclohexan und 8 g Triäthylamin in Methanol.

Farblose Kristalle. Schmp. 134—137°C.

Beispiel 44

5-Hydroxy-1-methyl-5-(4-methyl-3-sulfamoylphenyl)-2-oxopyrrolidin bzw. 4-(4-Methyl-3-sulfamoylphenyl)-4-oxobutansäure-N-methylamid

a) 10 g 4-(4-Methylphenyl)-4-oxobutansäure werden in 100 ml 100%ige Salpetersäure (d = 1,52) bei —15°C eingetragen, 10 Min. bei angegebener Temperatur gerührt und die Kristalline 4-(4-Chlor-3-nitrophenyl)-4-oxo-butansäure abfiltriert. Schmp. 143—145°C (aus Wasser-Äthanol).

b) 35 g 4-(4-Chlor-3-nitrophenyl)-4-oxobutansäure werden in einem Gemisch aus 350 ml Methanol und 85 ml Dioxan mit Raney-Nickel und Wasserstoff bei 40 atm. und 50°C hydriert. Nach dem Einengen läßt man die 4-(3-Amino-4-methylphenyl)-4-oxobutansäure (Schmp. 102—105°C) in 350 ml Wasser kristallisieren.

c) 3,7 g 4-(3-Amino-4-methylphenyl)-4-oxobutansäure werden nach Zugabe von 33 ml 2 N HCl bei 0°C mit einer Lösung von 1,2 g Natriumnitrit in 6,5 ml Wasser diazotiert und sodann die Lösung portionsweise in eine Mischung aus 1,1 g $CuCl_2 \times 2\ H_2O$ und 20 ml mit $SO_2$ gesättigter Eisessiglösung gegossen. Nach 30 Min. Rührung bei 16—18°C versetzt man mit 100 ml $H_2O$, rührt weitere 30 Min. bei 0—5° und filtriert die kristalline 4-(3-Chlorsulfonyl-4-methylphenyl)-4-oxobutansäure (Schmp. 116—119°C).

d) 10 g 4-(3-Chlorsulfonyl-4-methylphenyl)-4-oxobutansäure werden in 70 ml flüss. Ammoniak eingetragen. Nach dem Verdampfen bei Raumtemperatur nimmt man den Rückstand mit 70 ml Wasser auf, stellt mit 2 N HCl auf pH 1 bis 2 und filtriert die kristalline 4-(4-Methyl-3-sulfamoylphenyl)-4-oxobutansäure, Schmp. 165°C.

e) 9,4 g 4-(4-Methyl-3-sulfamoylphenyl)-4-oxobutansäure werden analog der in Beispiel 1 angegebenen Vorschrift unter Verwendung von 64 ml abs. THF, 6,7 ml Triäthylamin, 4,7 ml Chlorameisensäureäthylester und 90 ml 40%iger wäßriger Methylaminlösung zu 5-Hydroxy-1-methyl-5-(4-methyl-3-sulfamoylphenyl)-2-oxopyrrolidin umgesetzt.

Farblose Kristalle aus wenig Acetonitril, Schmp. 124°C.

Beispiel 45

5-Hydroxy-1-methyl-2-oxo-5-(3-di-n-propylsulfamoylphenyl)pyrrolidin bzw. 4-Oxo-4-(3-di-n-propylsulfamoylphenyl)-butansäure-N-methylamid

a) 4-(3-Chlorsulfonylphenyl)-4-oxobutansäure erhält man analog der in Beispiel 44 c) angegebenen Vorschrift aus 4-(3-Amino-phenyl)-4-oxobutansäure (farblose Kristalle, Schmp. 136—138°C) und wird analog Beispiel 15 a) mit Di-n-propylamin in 4-Oxo-4-(3-di-n-propylsulfamoylphenyl)-butansäure (Schmp. 73—78°C) übergeführt.

b) 5-Hydroxy-1-methyl-2-oxo-5-(3-di-n-propylsulfamoylphenyl)-pyrrolidin erhält man analog der in Beispiel 1 angegebenen Vorschrift aus 6,8 g 4-Oxo-4-(3-di-n-propylsulfamoylphenyl)-butansäure unter Verwendung von 100 ml abs. THF, 3,2 ml Triäthylamin, 2,2 ml Chlorameisensäureäthylester und 100 ml 40%ige wäßrige Methylaminlösung.

Farblose Kristalle, Schmp. 58—60°C.

Beispiel 46

5-(4-Chlor-3-di-n-propylsulfamoylphenyl)-5-hydroxy-1-methyl-2-oxopyrrolidin bzw. 4-(4-Chlor-3-di-n-propylsulfamoylphenyl)-4-oxobutansäure-N-methylamid

erhält man analog Beispiel 41 aus 5-(4-Chlor-3-di-n-propylsulfamoylphenyl)-2,3-dihydro-2-oxofuran und 40%iger wäßriger Methylaminlösung.

Farblose Kristalle, Schmp. 112—114°C.

Das verwendete 5-(4-Chlor-3-di-n-propylsulfamoyl-phenyl)-2,3-dihydro-2-oxofuran (Schmp. 118—121°C) wird analog Beispiel 41 a) aus 4-(4-Chlor-3-di-n-propylsulfamoylphenyl)-4-oxobutansäure und Acetanhydrid dargestellt.

Beispiel 47

5-(4-Chlor-3-methylsulfamoylphenyl)-5-hydroxy-1-methyl-2-oxopyrrolidin

Zu einer Mischung aus 6,2 g 4-(4-Chlor-3-chlorsulfonylphenyl)-4-oxobutansäure und 2,2 g Chlorameisensäureäthylester in 20 ml abs. Tetrahydrofuran tropft man rasch 2,1 g Triäthylamin so zu, daß die Innentemperatur zwischen —5 und —10°C gehalten wird. Man rührt ca. 5 bis 10 Min. bei —5 bis 0°C und tropft sodann bei —5°C zum Reaktionsgemisch rasch 1,55 ml einer 4%igen Lösung von gasförmigen Methylamin in Tetrahydrofuran. Man rührt 15 bis 20 Min. bei 10 bis 15°, gießt das Reaktionsgemisch in Wasser und extrahiert 2 x mit je 80 ml Essigester. Die organische Phase wird 1 x mit 50 ml 0° kalter 0,01 N Salzsäure und 1 x mit kalter Bicarbonatlösung gewaschen, über $Na_2SO_4$ bei 0—10°C unter magnet. Rührung 1 hr getrocknet und am Rotavapor eingeengt (Badtemperatur $\leqslant$30°). Man erhält das 5-(4-Chlor-3-chlorsulfonylphenyl)-5-hydroxy-1-methyl-2-oxopyrrolidin zumeist als amorphen Rückstand. Nach Zugabe von 20 ml einer 40%igen wäßrigen Methylaminlösung (Kühlung) rührt man über Nacht bei Raumtemperatur, engt ein und läßt unter Wasser kristallisieren.

Farblose Kristalle, Schmp. 112—114°C (aus Äthanol).

Beispiel 48

5-(4-Chlor-3-di-n-propylsulfamoyl-phenyl)-5-hydroxy-1-methyl-2-oxopyrrolidin

erhält man durch Rühren des in Beispiel 47 dargestellten 5-(4-Chlor-3-chlorsulfonylphenyl)-5-hydroxy-1-methyl-2-oxopyrrolidin in einer Lösung von 5 g Di-n-propylamin in 40 ml Methanol bei Raumtemperatur über 14 Stunden. Nach dem Einengen arbeitet man den Rückstand analog der in Beispiel 1 angegebenen Vorschrift auf.

Farblose Kristalle, Schmp. 111—113°C.

## Patentansprüche

1. Benzolsulfonamidderivate der allgemeinen Formel I

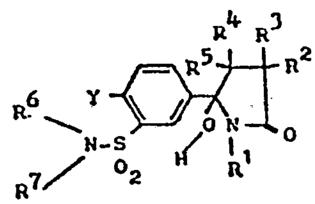

I

bzw. ihrer tautomeren Form I a

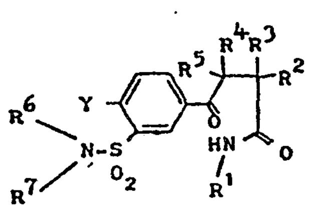

in der $R^1$ Wasserstoff, Alkyl mit 1 bis 4 C-Atomen oder Alkenyl mit 2 bis 4 C-Atomen, wobei der Alkylrest auch eine Methoxygruppe tragen kann, Cycloalkyl mit 3 bis 5 Ringgliedern, Benzyl, $R^2$ bis $R^6$ Wasserstoff oder einen Alkylrest mit 1 bis 4 C-Atomen, $R^7$ Wasserstoff, Alkyl mit 1 bis 10 C-Atomen, wobei der Alkylrest auch 1 bis 2 Methoxy- oder Äthoxygruppen oder eine Äthylendioxy- oder eine Propylendioxygruppe tragen kann, Alkenyl mit 3 bis 5 C-Atomen, gegebenenfalls durch eine Methylgruppe substituiertes Cycloalkyl mit 3 bis 12 Ringgliedern, Cycloalkylalkyl mit 5 bis 6 Ringgliedern und mit 1 oder 2 C-Atomen im Alkylteil, Phenylalkyl mit 1 bis 2 C-Atomen im Alkylteil, wobei der Phenylrest einfach oder zweifach substituiert sein und als Substituenten Methyl, Methoxy, oder Chlor traken kann, $R^6$ und $R^7$ auch gemeinsam mit dem N-Atom einen gesättigten 5 bis 6 gliedrigen heterocyclischen Ring, Y Halogen, Wasserstoff, Trifluormethyl oder Methyl bedeutet.

2. 5-(4-Chlor-3-sulfamoylphenyl)-5-hydroxy-1-methyl-2-oxopyrrolidin bzw. 4-(4-Chlor-3-sulfamoylphenyl)-4-oxobutansäure-methylamid.

3. 5-(4-Brom-3-sulfamoylphenyl)-5-hydroxy-1-methyl-2-oxopyrrolidin bzw. 4-(4-Brom-3-sulfamoylphenyl)-4-oxobutansäure-methylamid.

4. 5-(4-Chlor-3-methylsulfamoylphenyl)-5-hydroxy-1-methyl-2-oxopyrrolidin bzw. 4-(4-Chlor-3-methylsulfamoylphenyl)-4-oxobutansäure-methylamid.

5. 5-(4-Chlor-3-propylsulfamoylphenyl)-5-hydroxy-1-methyl-2-oxopyrrolidin bzw. 4-(4-Chlor-3-propylsulfamoylphenyl)-4-oxobutansäure-methylamid.

6. 5-(3-Allylsulfamoyl-4-chlorphenyl)-5-hydroxy-1-methyl-2-oxopyrrolidin bzw. 4-(3-Allylsulfamoyl-4-chlorphenyl)-4-oxobutansäure-methylamid.

7. 5-(4-Chlor-3-isobutylsulfamoylphenyl)-5-hydroxy-1-methyl-2-oxopyrrolidin bzw. 4-(4-Chlor-3-isobutylsulfamoylphenyl)-4-oxobutansäure-methylamid.

8. 5-(4-Chlor-3-sek.butylsulfamoylphenyl)-5-hydroxy-1-methyl-2-oxopyrrolidin bzw. 4-(4-Chlor-3-sek.butylsulfamoylphenyl)-4-oxobutansäure-methylamid.

9. 5-(4-Chlor-3-hexylsulfamoylphenyl)-5-hydroxy-1-methyl-2-oxopyrrolidin bzw. 4-(4-Chlor-3-hexylsulfamoylphenyl)-4-oxobutansäure-methylamid.

10. 5-(4-Chlor-3-dimethylsulfamoylphenyl)-5-hydroxy-1-methyl-2-oxopyrrolidin bzw. 4-(4-Chlor-3-dimethyl-sulfamoylphenyl)-4-oxobutansäure-methylamid.

11. 5-(4-Chlor-3-dipropylsulfamoylphenyl)-5-hydroxy-1-methyl-2-oxopyrollidin bzw. 4-(4-Chlor-3-dipropyl-sulfamoylphenyl)-4-oxobutansäure-methylamid.

12. Verfahren zur Herstellung von Verbindungen gemäß Anspruch 1, dadurch gekennzeichnet, daß man

a) Verbindungen der allgemeinen Formel II

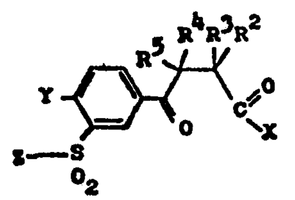

II

worin Z ein Halogenatom oder die $NR^6R^7$-Gruppe und X eine leaving group bedeutet und $R^2$ bis $R^7$ sowie Y die in Anspruch 1 angegebene Bedeutung besitzen, mit einem Amin der Formel III

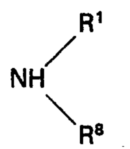

III

worin $R^1$ die in Anspruch 1 angegebene Bedeutung hat und $R^8$ Wasserstoff oder einen Benzyl- oder Diphenylmethylreste bedeutet, die gegebenenfalls durch Methoxygruppen substituiert sein können, umsetzt und, falls $R^8$ nicht Wasserstoff bedeutet, die erhaltenen Verbindungen der Hydrogenolyse oder Hydrolyse unterwirft oder

b) Verbindungen der allgemeinen Formel IV

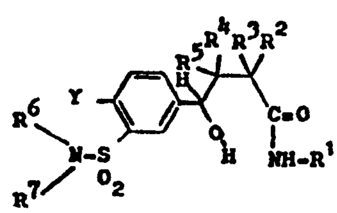

IV

worin $R^1$ bis $R^7$ und Y die angegebene Bedeutung besitzen, mit einem Oxidationsmittel behandelt, oder

c) Verbindungen der allgemeinen Formel V

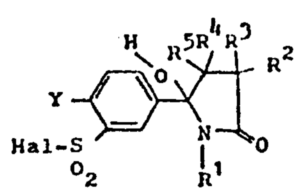

V

worin Y und $R^1$ bis $R^5$ die obige Bedeutung besitzen und Hal für Halogen steht, mit Ammoniak oder einem Amin der allgemeinen Formel VI

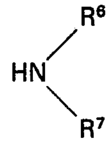

VI

worin $R^6$ und $R^7$ die angegebene Bedeutung besitzen, umsetzt oder

d) Verbindungen der allgemeinen Formel VII

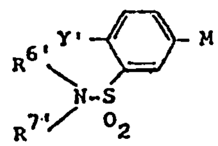

VII

worin M für Li oder MgHal steht, Y' die Bedeutung von Y hat, jedoch nicht für Brom oder Jod steht, und $R^{6'}$ und $R^{7'}$ mit Ausnahme von Wasserstoff die Bedeutung von $R^6$ und $R^7$ besitzen oder für ein Metallkation stehen, mit Succinimidderivaten der allgemeinen Formel VIII

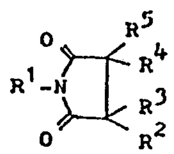

VIII

worin $R^1$ bis $R^5$ die angegebene Bedeutung besitzen, umsetzt und die erhaltenen Verbindungen nachfolgend mit einer Säure behandelt, oder

e) Verbindungen der allgemeinen Formel IX

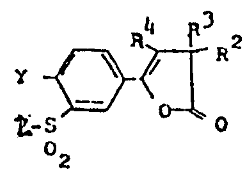

IX

mit einem Amin der Formel III umsetzt, wobei $R^1$ bis $R^4$, Z und Y die angegebene Bedeutung besitzen und gegebenenfalls die nach Weg a) bis c) erhaltenen Verbindungen der allgemeinen Formel I, worin $R^6$ und/oder $R^7$ Wasserstoff bedeutet, alkyliert.

13. Pharmazeutische Präparate, gekennzeichnet durch einen Gehalt an Verbindungen gemäß Anspruch 1.

14. Verbindungen gemäß Anspruch 1 zur Anwendung bei der Behandlung von hypertonen Zuständen.

**Claims**

1. Benzenesulfonamide derivatives of the general formula I

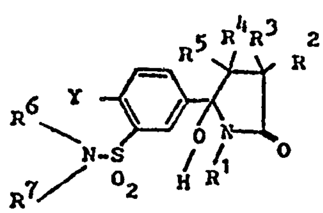

I

and in their tautomeric form Ia

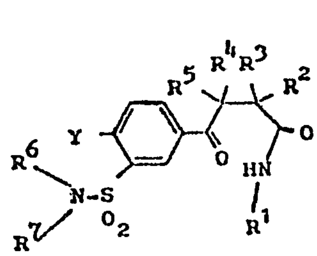

Ia

in which $R^1$ denotes hydrogen, alkyl or alkenyl with 1 to 4 C atoms, it also being possible for the alkyl radical to carry a methoxy group, cycloalkyl with 3 to 5 ring members or benzyl, $R^2$ to $R^6$ denote hydrogen or an alkyl radical with 1 to 4 C atoms, $R^7$ denotes hydrogen, alkyl with 1 to 10 C atoms, it also being possible for the alkyl radical to carry 1 to 2 methoxy or ethoxy groups or an ethylenedioxy or propylenedioxy group, alkenyl with 3 to 5 C atoms, cycloalkyl with 3 to 12 ring members, which is optionally substituted by a methyl group, cycloalkylalkyl with 5 or 6 ring members and with 1 or 2 C atoms in the alkyl part, phenylalkyl with 1 to 2 C atoms in the alkyl part, it being possible for the phenyl radical to be monosubstituted or disubstituted and to carry methyl, methoxy or chlorine as substituents, or $R^6$ and $R^7$, together with the N atom, also denote a saturated 5-membered to 6-membered heterocyclic ring and Y denotes halogen, hydrogen, trifluoromethyl or methl.

2. 5-(4-Chloro-3-sulfamoylphenyl)-5-hydroxy-1-methyl-2-oxo-pyrrolidine or 4-(4-chloro-3-sulfamoylphenyl)-4-oxobutanoic acid methylamide.

3. 5-(4-Bromo-3-sulfamoylphenyl)-5-hydroxy-1-methyl-2-oxo-pyrrolidine or 4-(4-bromo-3-sulfamoylphenyl)-4-oxobutanoic acid methylamide.

4. 5-(4-Chloro-3-methylsulfamoylphenyl)-5-hydroxy-1-methyl-2-oxopyrrolidine or 4-(4-chloro-3-methylsulfamoylphenyl)-4-oxo-butanoic acid methylamide.

5. 5-(4-Chloro-3-propylsulfamoylphenyl)-5-hydroxy-1-methyl-2-oxopyrrolidine or 4-(4-chloro-3-propylsulfamoylphenyl)-4-oxo-butanoic acid methylamide.

6. 5-(3-Allylsulfamoyl-4-chlorophenyl)-5-hydroxy-1-methyl-2-oxopyrrolidine or 4-(3-allylsulfamoyl-4-chlorophenyl)-4-oxobutanoic acid methylamide.

7. 5-(4-Chloro-3-isobutylsulfamoylphenyl)-5-hydroxy-1-methyl-2-oxopyrrolidine or 4-(4-chloro-3-isobutylsulfamoylphenyl)-4-oxobutanoic acid methylamide.

8. 5-(4-Chloro-3-sec.-butylsulfamoylphenyl)-5-hydroxy-1-methyl-2-oxopyrrolidine or 4-(4-chloro-3-sec.-butylsulfamoylphenyl)-4-oxobutanoic acid methylamide.

9. 5-(4-Chloro-3-hexylsulfamoylphenyl)-5-hydroxy-1-methyl-2-oxopyrrolidine or 4-(4-chloro-3-hexylsulfamoylphenyl)-4-oxo-butanoic acid methylamide.

10. 5-(4-Chloro-3-dimethylsulfamoylphenyl)-5-hydroxy-1-methyl-2-oxopyrrolidine or 4-(4-chloro-3-dimethylsulfamoylphenyl)-4-oxobutanoic acid methylamide.

11. 5-(4-Chloro-3-dipropylsulfamoylphenyl)-5-hydroxy-1-methyl-2-oxopyrrolidine or 4-(4-chloro-3-dipropylsulfamoylphenyl)-4-oxobutanoic acid methylamide.

12. Process for the manufacture of compounds as claimed in Claim 1, which comprises

a) reacting compounds of the general formula II

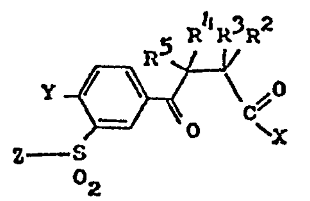

II

wherein Z denotes a halogen atom or the group $NR^6R^7$, X denotes a leaving group and $R^2$ to $R^7$ and Y have the meaning indicated in Claim 1, with an amine of the formula III

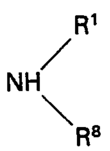

III

wherein $R^1$ has the meaning indicated in Claim 1 and $R^8$ denotes hydrogen or a benzyl or diphenylmethyl radical, which can be optionally substituted by methoxy groups, and, if $R^8$ does not denote hydrogen, subjecting the resulting compounds to hydrogenolysis or hydrolysis, or

b) treating compounds of the general formula IV

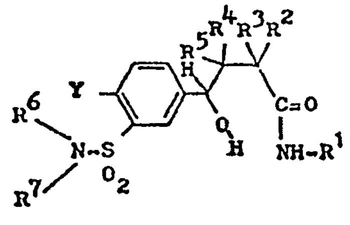

IV

wherein $R^1$ to $R^7$ and Y have the meaning indicated, with an oxidizing agent, or

c) reacting compounds of the general formula V

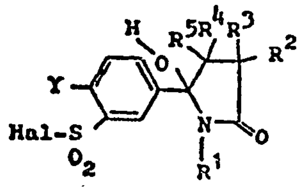

V

wherein Y and $R^1$ to $R^5$ have the above meaning and Hal represents halogen, with ammonia or an amine of the general formula VI

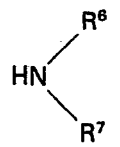

VI

wherein $R^6$ and $R^7$ have the meaning indicated, or

d) reacting compounds of the general formula VII

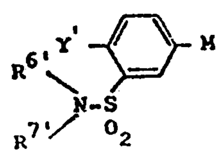

VII

wherein M represents Li or MgHal, Y has the above meaning, but does not represent bromine or iodine, and $R^{6'}$ and $R^{7'}$ have the meaning of $R^6$ and $R^7$, with the exception of hydrogen, or represent a metal cation, with succinimide derivatives of the general formula VIII

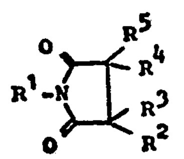

VIII

wherein $R^1$ to $R^5$ have the meaning indicated, and subsequently treating the resulting compounds with an acid, or

e) reacting compounds of the general formula IX

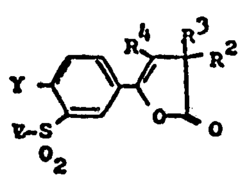

IX

wherein $R^1$ to $R^4$, Z and Y have the meaning indicated, with an amine of the formula III, and optionally alkylating compounds of the general formula I, wherein $R^6$ and/or $R^7$ denote hydrogen, obtained by routes a) to e).

13. Pharmaceutical formulations which contain compounds as claimed in Claim 1.

14. Compounds according to claim 1 for use in the treatment of hypertonic states.

**Revendications**

1. Dérivés de benzène sulfonamide de formule générale I

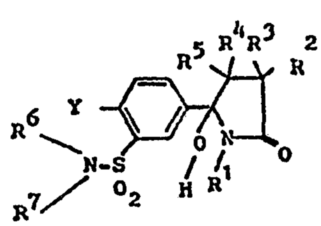

I

ou leur forme tautomère I a

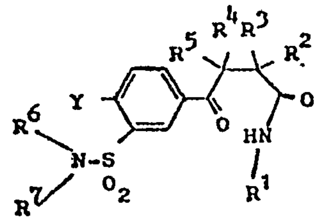

Ia

dans lesquelles $R^1$ représente l'hydrogène, un radical alkyle ayant de 1 à 4 atomes de carbone ou alcényle ayant de 2 à 4 atomes de carbone, le radical alkyle pouvant également porter un groupe méthoxy, un radical cycloalkyle ayant de 3 à 5 chaînons dans le cycle, ou un groupe benzyle; $R^2$ à $R^6$ représentent l'hydrogène ou un radical alkyle ayant de 1 à 4 atomes de carbone; $R^7$ représente l'hydrogène, un radical alkyle ayant de 1 à 10 atomes de carbone, le radical alkyle pouvant également porter 1 ou 2 groupes méthoxy ou éthoxy ou un groupe éthylène dioxy ou propylène dioxy, un radical alcényle ayant de 3 à 5 atomes de carbone, un radical cycloalkyle ayant de 3 à 12 chaînons dans le cycle, éventuellement substitué par un groupe méthyle, un radical cycloalkylalkyle ayant 5 ou 6 chaînons dans le cycle et ayant 1 ou 2 atomes de carbone dans le fragment alkyle, un radical phénylalkyle ayant 1 ou 2 atomes de carbone dans le fragment alkyle, le radical phényle pouvant être substitué une ou plusieurs fois par des substituants méthyle, méthoxy ou chlore; $R^6$ et $R^7$ représentent également ensemble avec l'atome d'azote un noyau hétérocyclique saturé à 5 à 6 chaînons; et Y représente un halogène ou un groupe trifluorométhyle ou méthyle.

2. 5-(4-chloro-3-sulfamoylphényl)-5-hydroxy-1-méthyl-2-oxopyrrolidine ou méthylamide de l'acide 4-(4-chloro-3-sulfamoylphényl)-4-oxobutanoïque.

3. 5-(4-bromo-3-sulfamoylphényl)-5-hydroxy-1-méthyl-2-oxopyrrolidine ou méthylamide de l'acide 4-(4-bromo-3-sulfamoylphényl)-4-oxobutanoïque.

4. 5-(4-chloro-3-méthylsulfamoylphényl)-5-hydroxy-1-méthyl-2-oxopyrrolidine ou méthylamide de l'acide 4-(4-chloro-3-méthylsulfamoylphényl)-4-oxobutanoïque.

5. 5-(4-chloro-3-propylsulfamoylphényl)-5-hydroxy-1-méthyl-2-oxopyrrolidine ou méthylamide de l'acide 4-(4-chloro-3-propylsulfamoylphényl)-4-oxobutanoïque.

6. 5-(3-allylsulfamoyl-4-chlorophényl)-5-hydroxy-1-méthyl-2-oxopyrrolidine ou méthylamide de l'acide 4-(3-allyl-sulfamoyl-4-chlorophényl)-4-oxobutanoïque.

7. 5-(4-chloro-3-isobutylsulfamoylphényl)-5-hydroxy-1-méthyl-2-oxopyrrolidine ou méthylamide de l'acide 4-(4-chloro-3-isobutylsulfamoylphényl)-4-oxobutanoïque.

8. 5-(4-chloro-3-sec.butylsulfamoylphényl)-5-hydroxy-1-méthyl-2-oxopyrrolidine ou méthylamide de l'acide 4-(4-chloro-3-sec.butylsulfamoylphényl)-4-oxobutanoïque.

9. 5-(4-chloro-3-hexylsulfamoylphényl)-5-hydroxy-1-méthyl-2-oxopyrrolidine ou méthylamide de l'acide 4-(4-chloro-3-hexylsulfamoylphényl)-4-oxobutanoïque.

10. 5-(4-chloro-3-diméthylsulfamoylphényl)-5-hydroxy-1-méthyl-2-oxopyrrolidine ou méthylamide de l'acide 4-(4-chloro-3-diméthylsulfamoylphényl)-4-oxobutanoïque.

11. 5-(4-chloro-3-dipropylsulfamoylphényl)-5-hydroxy-1-méthyl-2-oxopyrrolidine ou méthylamide de l'acide 4-(4-chloro-3-dipropylsulfamoylphényl)-4-oxobutanoïque.

12. Procédé de préparation des composés selon la revendication 1, caractérisé en ce que:

a) on fait réagir des composés de formule générale II

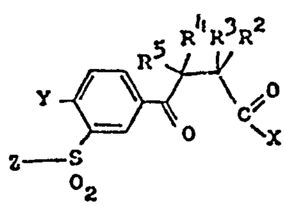

II

dans laquelle Z représente un atome d'halogène ou le groupe $NR^6R^7$ et X est un groupe éliminable et $R^2$ à $R^7$ ainsi que Y ont les significations indiquées dans la revendication 1, avec une amine de formule III

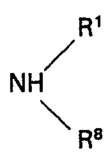

III

dans laquelle $R^1$ a la signification indiquée dans la revendication 1 et $R^8$ représente l'hydrogène ou un radical benzyle ou diphénylméthyle qui peut être éventuellement substiué par des groupes méthoxy,

et si $R^8$ ne représente pas l'hydrogène, on soumet les composés obtenus à une hydrogénolyse ou à une hydrolyse, ou

b) on traite des composés de formule générale IV

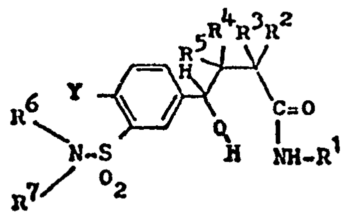

IV

dans laquelle $R^1$ à $R^7$ et Y ont les significations indiquées, par un oxydant, ou

c) on fait réagir des composés de formule générale V

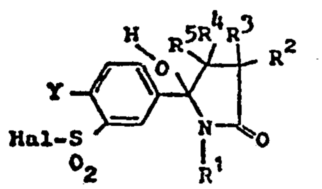

V

dans laquelle Y et $R^1$ à $R^5$ ont les significations indiquées ci-dessus et Hal représente un halogène, avec l'ammoniac ou une amine de formule générale VI

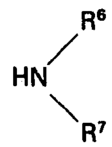

VI

dans laquelle $R^6$ et $R^7$ ont les significations indiquées, ou

d) on fait réagir des composés de formule générale VII

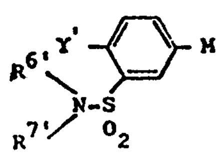

VII

dans laquelle M représente Li ou MgHal, Y' a la signification de Y, mais ne peut être ni le brome ni l'iode, et $R^{6'}$ et $R^{7'}$ ont les mêmes significations que $R^6$ et $R^7$ à l'exception de l'hydrogène ou représentent un cation métallique, avec des dérivés de succinimide de formule générale VIII

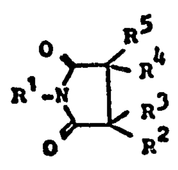

VIII

dans laquelle $R^1$ à $R^5$ ont les significations indiquées, et on traite ensuite les composés obtenus par un acide, ou

e) on fait réagir des composés de formule générale IX

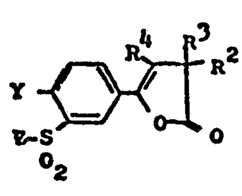

avec une amine de formule III, $R^1$ à $R^4$, Z et Y ayant les significations indiquées, et éventuellement on

alkyle les composés de formule générale I dans laquelle $R^6$ et/ou $R^7$ représentent l'hydrogène obtenu selon les modes opératoires a) à c).

13. Préparations pharmaceutiques, caractérisées en ce qu'elles contiennent des composés selon la revendication 1.

14. Composés selon la revendication 1 pour utilisation dans le traitement des états hypertoniques.